(19) Europäisches Patentamt
European Patent Office
Office européen des brevets

(11) Veröffentlichungsnummer: **0 382 034 A1**

(12) **EUROPÄISCHE PATENTANMELDUNG**

(21) Anmeldenummer: **90101681.6**

(51) Int. Cl.[5]: **C07D 231/16, A01N 43/56, C07D 405/04**

(22) Anmeldetag: **28.01.90**

(30) Priorität: **09.02.89 DE 3903799**

(43) Veröffentlichungstag der Anmeldung: **16.08.90 Patentblatt 90/33**

(84) Benannte Vertragsstaaten: **BE CH DE DK FR GB IT LI NL**

(71) Anmelder: **BAYER AG**

**D-5090 Leverkusen 1 Bayerwerk(DE)**

(72) Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Fischer, Reiner, Dr.**
**Nelly-Sachs-Strasse 23**
**D-4019 Monheim 2(DE)**
Erfinder: **Marhold, Albrecht, Dr.**
**Carl-Duisberg-Strasse 329**
**D-5090 Leverkusen(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Heiderweg 53**
**D-4000 Düsseldorf 31(DE)**

(54) **N-Aryl-Stickstoffheterocyclen.**

(57) Die Erfindung betrifft neue N-Aryl-Stickstoffheterocyclen der allgemeinen Formel (I),

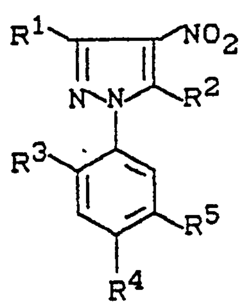

(I)

in welcher
$R^1$ für Wasserstoff, Alkyl oder Halogenalkyl steht,
$R^2$ für Wasserstoff, Alkyl oder Halogenalkyl steht,
$R^3$ für Wasserstoff oder Halogen steht und entweder
$R^4$ für Cyano, Nitro, Alkyl, Alkenyl, Alkinyl, Alkoxy, Halogenalkyl oder Halogenalkoxy steht und
$R^5$ für Halogen, für einen Rest -O-$R^6$ oder für einen Rest -S-$R^7$ steht oder
$R^4$ und $R^5$ gemeinsam für einen gegebenenfalls substituierten Dioxyalkylenrest stehen,
mehrere Verfahren zu ihrer Herstellung und ihre Verwendung als Herbizide.

EP 0 382 034 A1

## N-Aryl-Stickstoffheterocyclen

Die Erfindung betrifft neue N-Aryl-Stickstoffheterocyclen, mehrere Verfahren zu ihrer Herstellung und ihre Verwendung als Herbizide.

Es ist bekannt, daß bestimmte N-Aryl-Stickstoffheterocyclen, wie beispielsweise die Verbindung 1-(2-Chlor-4-trifluormethylphenyl)-5-methyl-4-nitropyrazol herbizide Eigenschaften besitzen (vgl. z.B. EP-A 200 872).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin ist die Verbindung 1-(3,4-Dimethoxyphenyl)-4-nitropyrazol bekannt (vgl. z.B. Farmaco Ed. Sci. 19, 459-473 [1964]). Über eine herbizide Wirksamkeit dieser Verbindung ist nichts bekannt.

Es wurden neue N-Aryl-Stickstoffheterocyclen der allgemeinen Formel (I),

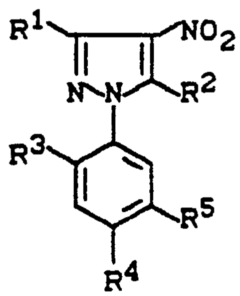

(I)

in welcher

$R^1$ für Wasserstoff, Alkyl oder Halogenalkyl steht,

$R^2$ für Wasserstoff, Alkyl oder Halogenalkyl steht,

$R^3$ für Wasserstoff oder Halogen steht und entweder

$R^4$ für Cyano, Nitro, Alkyl, Alkenyl, Alkinyl, Alkoxy, Halogenalkyl oder Halogenalkoxy steht und

$R^5$ für Halogen, für einen Rest $-O-R^6$ oder für einen Rest $-S-R^7$ steht oder

$R^4$ und $R^5$ gemeinsam für einen gegebenenfalls substituierten Dioxyalkylenrest stehen,

wobei

$R^6$ für Wasserstoff, für jeweils gegebenenfalls substituiertes Alkyl, Alkenyl, Alkinyl oder Cycloalkyl steht oder für einen Rest $-\underset{\underset{O}{\|}}{C}-R^8$

oder einen Rest $-SO_2-R^9$ steht,

$R^7$ für Wasserstoff, für jeweils gegebenenfalls substituiertes Alkyl, Alkenyl, Alkinyl oder Cycloalkyl steht oder für einen Rest $-\underset{\underset{O}{\|}}{C}-R^8$

steht,

$R^8$ für Alkyl, Alkoxy oder Halogenalkyl steht und

$R^9$ für Alkyl, Halogenalkyl oder für gegebenenfalls substituiertes Aryl steht,

wobei jedoch die Verbindung 1-(3,4-Dimethoxyphenyl)-4-nitropyrazol ausgenommen ist,

gefunden.

Weiterhin wurde gefunden, daß man die neuen N-Aryl-Stickstoffheterocyclen der allgemeinen Formel (I),

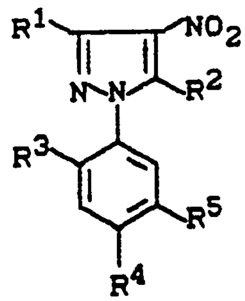

(I)

in welcher
$R^1$ für Wasserstoff, Alkyl oder Halogenalkyl steht,
$R^2$ für Wasserstoff, Alkyl oder Halogenalkyl steht,
$R^3$ für Wasserstoff oder Halogen steht und entweder
$R^4$ für Cyano, Nitro, Alkyl, Alkenyl, Alkinyl, Alkoxy, Halogenalkyl oder Halogenalkoxy steht und
$R^5$ für Halogen, für einen Rest $-O-R^6$ oder für einen Rest $-S-R^7$ steht oder
$R^4$ und $R^5$ gemeinsam für einen gegebenenfalls substituierten Dioxyalkylenrest stehen,
wobei
$R^6$ für Wasserstoff, für jeweils gegebenenfalls substituiertes Alkyl, Alkenyl, Alkinyl oder Cycloalkyl steht oder für einen Rest $-\underset{\underset{O}{\|}}{C}-R^8$
oder einen Rest $-SO_2-R^9$ steht,
$R^7$ für Wasserstoff, für jeweils gegebenenfalls substituiertes Alkyl, Alkenyl, Alkinyl oder Cycloalkyl steht oder für einen Rest $-\underset{\underset{O}{\|}}{C}-R^8$
steht,
$R^8$ für Alkyl, Alkoxy oder Halogenalkyl steht und
$R^9$ für Alkyl, Halogenalkyl oder für gegebenenfalls substituiertes Aryl steht,
wobei jedoch die Verbindung 1-(3,4-Dimethoxyphenyl)-4-nitropyrazol ausgenommen ist,
nach einem der im Folgenden beschriebenen Verfahren erhält:

(a) Man erhält N-Aryl-Stickstoffheterocyclen der Formel (I),

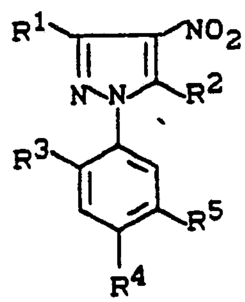

(I)

in welcher
$R^1$, $R^2$, $R^3$, $R^4$ und $R^5$ die oben angegebene Bedeutung haben,
wenn man 4-unsubstituierte 1-Phenylpyrazole der Formel (II),

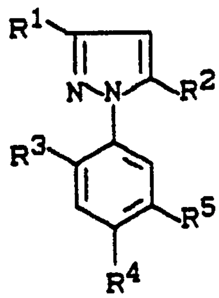

(II)

in welcher
$R^1$, $R^2$, $R^3$, $R^4$ und $R^5$ die oben angegebene Bedeutung haben,
mit einem Nitrierungsmittel gegebenenfalls in Gegenwart eines Verdünnungsmittels und gegebenenfalls in Gegenwart eines Reaktionshilfsmittels umsetzt;

(b) man erhält N-Aryl-Stickstoffheterocyclen der Formel (Ia),

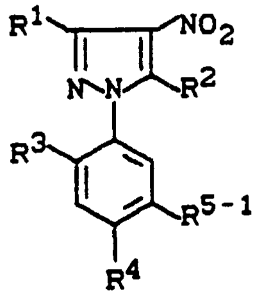

(Ia)

in welcher
$R^{5-1}$ für einen Rest $-O-R^{6-1}$ oder für einen Rest $-S-R^{7-1}$ steht, wobei
$R^{6-1}$ für jeweils gegebenenfalls substituiertes Alkyl, Alkenyl, Alkinyl oder Cycloalkyl steht, für einen Rest $-\underset{\underset{O}{\|}}{C}-R^8$ oder für einen Rest $-SO_2-R^9$ steht und
$R^{7-1}$ für jeweils gegebenenfalls substituiertes Alkyl, Alkenyl, Alkinyl oder Cycloalkyl steht oder für einen Rest $-\underset{\underset{O}{\|}}{C}-R^8$ steht und wobei
$R^1$, $R^2$, $R^3$, $R^4$, $R^8$ und $R^9$ die oben angegebene Bedeutung haben,
alternativ auch wenn man N-Aryl-Stickstoffheterocyclen der Formel (Ib),

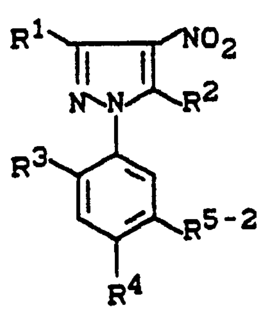

(Ib)

in welcher
$R^{5-2}$ für Hydroxy oder Mercapto steht und
$R^1$, $R^2$, $R^3$ und $R^4$ die oben angegebene Bedeutung haben,

($\alpha$) mit Alkylierungsmitteln der Formel (IIIa),

$R^{10}-E^1$ (IIIa)

in welcher

$R^{10}$ für jeweils gegebenenfalls substituiertes Alkyl, Alkenyl, Akinyl oder Cycloalkyl steht und

$E^1$ für eine elektronenanziehende Abgangsgruppe steht oder

(ß) mit Acylierungsmitteln der Formel (IIIb),

$R^8-\underset{\overset{\|}{O}}{C}-E^2$ (IIIb)

in welcher

$E^2$ für eine elektronenanziehende Abgangsgruppe steht und

$R^8$ die oben angegebene Bedeutung hat und

(γ) mit Sulfonylierungsmitteln der Formel (IIIC),

$R^9-SO_2-E^3$ (IIIc)

in welcher

$E^3$ für eine elektronenanziehende Abgangsgruppe steht und

$R^9$ die oben angegebene Bedeutung hat,

jeweils gegebenenfalls in Gegenwart eines Verdünnungsmittels und gegebenenfalls in Gegenwart eines Reaktionshilfsmittels umsetzt;

(c) man erhält N-Aryl-Stickstoffheterocyclen der Formel (Ic),

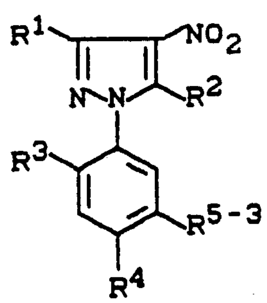

(Ic)

in welcher

$R^{5-3}$ für einen Rest $-O-R^{10}$ oder für einen Rest $-S-R^{10}$ steht, wobei

$R^{10}$ für jeweils gegebenenfalls substituiertes Alkyl, Alkenyl, Alkinyl oder Cycloalkyl steht und wobei

$R^1$, $R^2$, $R^3$ und $R^4$ die oben angegebene Bedeutung haben,

alternativ auch, wenn man N-Aryl-Stickstoffheterocyclen der Formel (Id),

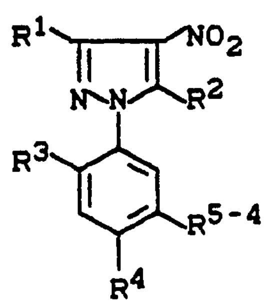

(Id)

in welcher

$R^{5-4}$ für Halogen steht und

$R^1$, $R^2$, $R^3$ und $R^4$ die oben angegebene Bedeutung haben,

mit Alkoholen oder Thiolen der Formel (IV),

$R^{5-3}-H$ (IV)

in welcher

$R^{5-3}$ die oben angegebene Bedeutung hat,

gegebenenfalls in Gegenwart eines Verdünnungsmittels und gegebenenfalls in Gegenwart eines Reaktionshilfsmittels umsetzt;

man erhält N-Aryl-Stickstoffheterocyclen der Formel (Ie),

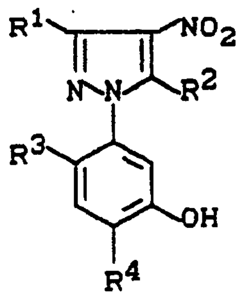

(Ie)

in welcher
$R^1$, $R^2$, $R^3$ und $R^4$ die oben angegebene Bedeutung haben,
alternativ auch, wenn man

(d) N-Aryl-Stickstoffheterocyclen der Formel (If),

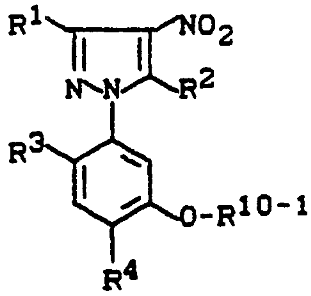

(If)

in welcher
$R^{10-1}$ für Allyl oder für Benzyl steht,
entweder für den Fall, daß $R^{10-1}$ für Benzyl steht mit Wasserstoff in Gegenwart eines geeigneten Hydrierkatalysators oder für den Fall, daß $R^{10-1}$ für Allyl steht mit verdünnten Säuren in Gegenwart eines geeigneten Isomerisierungskatalysators sowie jeweils gegebenenfalls in Gegenwart eines Verdünnungsmittels und gegebenenfalls in Gegenwart eines Reaktionshilfsmittels umsetzt oder wenn man

(e) N-Aryl-Stickstoffheterocyclen der Formel (Ig),

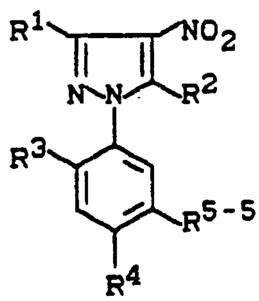

(Ig)

in welcher
$R^{5-5}$ für einen Rest $-O-\underset{\underset{O}{\|}}{C}-R^8$
oder für einen Rest $-O-SO_2-R^9$ steht und
$R^1$, $R^2$, $R^3$ und $R^4$ die oben angegebene Bedeutung haben,
mit verdünnten Säuren oder Basen gegebenenfalls in Gegenwart eines Verdünnungsmittels umsetzt;

(f) man erhält N-Aryl-Stickstoffheterocyclen der Formel (Ih),

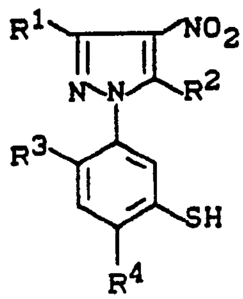

(Ih)

in welcher
$R^1$, $R^2$, $R^3$ und $R^4$ die oben angegebene Bedeutung haben,
alternativ auch, wenn man N-Arylstickstoffheterocyclen der Formel (Id),

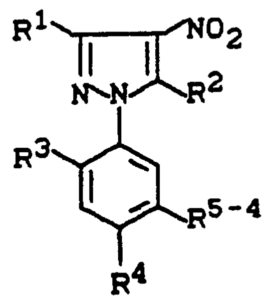

(Id)

in welcher
$R^{5-4}$ für Halogen steht und
$R^1$, $R^2$, $R^3$ und $R^4$ die oben angegebene Bedeutung haben,
mit Natriumhydrogensulfid gegebenenfalls in Gegenwart eines Verdünnungsmittels und gegebenenfalls in Gegenwart eines Reaktionshilfsmittels umsetzt.

Schließlich wurde gefunden, daß die neuen N-Aryl-Stickstoffheterocyclen der Formel (I) herbizide Eigenschaften besitzen.

Überraschenderweise zeigen die erfindungsgemäßen N-Aryl-Stickstoffheterocyclen der allgemeinen Formel (I) neben einer erheblich besseren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Technik bekannten N-Aryl-Stickstoffheterocyclen, wie beispielsweise die Verbindung 1-(2-Chlor-4-trifluormethylphenyl)-5-methyl-4-nitropyrazol, welche chemisch und wirkungsmäßig naheliegende Verbindungen sind.

Die erfindungsgemäßen N-Aryl-Stickstoffheterocyclen sind durch die Formel (I) allgemein definiert.

Bevorzugt sind Verbindungen der Formel (I), bei welchen
$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^2$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^3$ für Wasserstoff, Fluor, Chlor oder Brom steht und entweder
$R^4$ für Cyano, Nitro, für jeweils geradkettiges oder verzweigtes Alkyl oder Alkoxy mit jeweils 1 bis 4 Kohlenstoffatomen, für jeweils geradkettiges oder verzweigtes Alkenyl oder Alkinyl mit jeweils 2 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen steht und
$R^5$ für Fluor, Chlor, Brom oder für einen Rest -O-$R^6$ oder für einen Rest -S-$R^7$ steht oder
$R^4$ und $R^5$ gemeinsam für einen gegebenenfalls einfach oder mehrfach, gleich oder verschieden durch Fluor, Chlor, Brom oder durch geradkettiges oder verzweig tes Alkyl mit 1 bis 4 Kohlenstoffatomen substituierten Dioxyalkylenrest mit 1 bis 3 Kohlenstoffatomen stehen, wobei
$R^6$ für Wasserstoff, für jeweils geradkettiges oder verzweigtes Alkyl mit 1 bis 8 Kohlenstoffatomen, Alkenyl

mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Alkoxyalkoxyalkoxyalkyl, Bis-[Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen:

Halogen sowie jeweils geradkettiges oder ver zweigtes Alkyl oder Alkoxy mit jeweils 1 bis 4 Kohlenstoffatomen;

$R^6$ weiterhin für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^6$ ferner für einen Rest $-\underset{\underset{O}{\|}}{C}-R^8$

oder für einen Rest $-SO_2-R^9$ steht,

$R^7$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Alkoxyalkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen:

Halogen sowie jeweils geradkettiges oder verzweigtes Alkyl oder Alkoxy mit jeweils 1 bis 4 Kohlenstoffatomen;

$R^7$ weiterhin für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halo genalkyl, Halogenalkoxy oder Halogenalkylthio mit jeweils 1 bis 4 Kohlenstoffatomen und 1 bis 9 gleichen oder verschiedenen Halogenatomen;

$R^7$ ferner für einen Rest $-\underset{\underset{O}{\|}}{C}-R^8$

steht,

$R^8$ für jeweils geradkettiges oder verzweigtes Alkyl, Alkoxy oder Halogenalkyl mit jeweils 1 bis 6 Kohlenstoffatomen und im Fall des Halogenalkyl mit 1 bis 13 gleichen oder verschiedenen Halogenatomen steht und

$R^9$ für jeweils geradkettiges oder verzweigtes Alkyl oder Halogenalkyl mit jeweils 1 bis 6 Kohlenstoffatomen und im Fall des Halogenalkyl mit 1 bis 13 gleichen oder verschiedenen Halogenatomen steht und außerdem für gegebenenfalls einfach oder mehrfach, gleich oder verschieden substituiertes Phenyl steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy, oder Halogenalkylthio mit jeweils 1 bis 4 Kohlenstoffatomen und 1 bis 9 gleichen oder verschiedenen Halogenatomen, wobei jedoch die Verbindung 1-(3,4-Dimethoxyphenyl)-4-nitropyrazol ausgenommen ist.

Besonders bevorzugt sind Verbindungen der Formel (I), bei welchen

$R^1$ für Wasserstoff, Methyl, Ethyl, n- oder i-Propyl, n-, i-, s- oder t-Butyl, Trifluormethyl, Difluormethyl, Fluormethyl, Trichlormethyl, Dichlormethyl oder Chlormethyl steht,

$R^2$ für Wasserstoff, Methyl, Ethyl, n- oder i-Propyl, n-, i-, s- oder t-Butyl, Trifluormethyl, Difluormethyl, Fluormethyl, Triohlormethyl, Dichlormethyl oder Chlormethyl steht,

$R^3$ für Wasserstoff, Fluor oder Chlor steht und entweder

$R^4$ für Cyano, Nitro, Methyl, Methoxy, Ethyl, Ethoxy, Vinyl, Allyl, Ethinyl, Propargyl, Trifluormethyl, Difluormethyl, Trifluormethoxy oder Difluormethoxy steht und

$R^5$ für Fluor oder Chlor, für einen Rest $-O-R^6$ oder für einen Rest $-S-R^7$ steht oder

$R^4$ und $R^5$ gemeinsam für einen gegebenenfalls ein- bis vierfach, gleich oder verschieden durch Fluor, Chlor oder Methyl substituierten Dioxyalkylenrest mit 1 oder 2 Kohlenstoffatomen stehen, wobei

$R^6$ für Wasserstoff, für Methyl, Ethyl, n- oder i-Propyl, n-, i-, s- oder t-Butyl, für Allyl, Propar gyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein-bis dreifach, gleich oder verschieden substituiertes Benzyl oder Phenylethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio;

$R^6$ ferner für einen Rest $-\underset{\underset{O}{\|}}{C}-R^8$

oder für einen Rest $-SO_2-R^9$ steht,

$R^7$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein-bis dreifach, gleich oder verschieden substituiertes Benzyl oder Phenylethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio oder

$R^7$ für einen Rest $-\underset{\underset{O}{\|}}{C}-R^8$

steht,

$R^8$ für jeweils geradkettiges oder verzweigtes Alkyl, Alkoxy oder Halogenalkyl mit jeweils 1 bis 3 Kohlenstoffatomen und im Fall des Halogenalkyl mit 1 bis 7 gleichen oder verschiedenen Halogenatomen steht und

$R^9$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach gleich oder verschieden substituiertes Phenyl steht, wobei als Substituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

wobei jedoch die Verbindung 1-(3,4-Dimethoxy-phenyl)-4-nitropyrazol ausgenommen ist.

Ganz besonders bevorzugt sind Verbindungen der Formel (I), bei welchen

$R^1$ für Wasserstoff, Methyl, Ethyl oder Trifluormethyl steht,

$R^2$ für Wasserstoff, Methyl, Ethyl oder Trifluormethyl steht,

$R^3$ für Wasserstoff oder Fluor steht und entweder

$R^4$ für Cyano, Nitro, Methyl, Methoxy, Trifluormethyl, Difluormethyl, Trifluormethoxy oder Difluormethoxy steht und

$R^5$ für Fluor oder für einen Rest $-O-R^6$ oder für einen Rest $-S-R^7$ steht oder

$R^4$ und $R^5$ gemeinsam für einen gegebenenfalls einfach oder zweifach durch Fluor, Chlor oder Methyl substituierten Dioxymethylenrest oder für einen gegebenenfalls ein- bis vierfach gleich oder verschieden durch Fluor, Chlor oder Methyl substituierten Dioxyethylenrest stehen, wobei

$R^6$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein- bis dreifach gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio;

$R^6$ ferner für einen Rest $-\underset{\underset{O}{\|}}{C}-R^8$

oder für einen Rest $-SO_2-R^9$ steht,

$R^7$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein- bis dreifach gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio oder

$R^7$ für einen Rest $-\underset{\underset{O}{\|}}{C}-R^8$

steht,

$R^8$ für jeweils geradkettiges oder verzweigtes Alkyl, Alkoxy oder Halogenalkyl mit jeweils 1 bis 3 Kohlenstoffatomen und im Fall des Halogenalkyl mit 1 bis 7 gleichen oder verschiedenen Halogenatomen steht und

$R^9$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach gleich oder verschieden substituiertes Phenyl steht, wobei als Substituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

wobei jedoch die Verbindung 1-(3,4-Dimethoxyphenyl)-4-nitropyrazol ausgenommen ist.

Insbesondere bevorzugt sind Verbindungen der Formel (I), bei welchen

$R^1$ für Methyl steht,

$R^2$ für Methyl steht,

$R^3$ für Wasserstoff oder Fluor steht und entweder
$R^4$ für Cyano, Methyl, Methoxy, Trifluormethyl oder Trifluormethoxy steht und
$R^5$ für Fluor oder für einen Rest $-O-R^6$ steht, wobei
$R^6$ für Wasserstoff, Methyl, Ethyl, n- oder i-Propyl, Methoxymethyl, Ethoxymethyl, Methoxyethyl, Ethoxyethyl oder für einen Rest $-SO_2-R^9$ steht, wobei
$R^9$ für Methyl, Ethyl, n- oder i-Propyl steht, oder
$R^4$ und $R^5$ gemeinsam für einen gegebenenfalls einfach oder zweifach durch Fluor, Chlor oder Methyl substituierten Dioxymethylenrest oder für einen gegebenenfalls ein- bis vierfach gleich oder verschieden durch Fluor, Chlor oder Methyl substituierten Dioxyethylenrest stehen.

Im einzelnen seien außer den bei den Herstellungsbeispielen genannten Verbindungen die folgenden N-Aryl-Stickstoffheterocyclen der allgemeinen Formel (I) genannt:

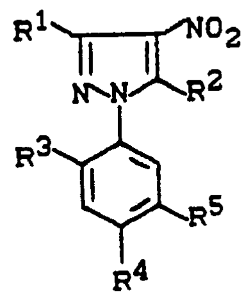

(I)

| $R^1$ | $R^2$ | $R^3$ | $R^4$ | $R^5$ |
|---|---|---|---|---|
| $CH_3$ | $CH_3$ | F | CN | $-O-CH_2-CH_2-OC_2H_5$ |
| $CH_3$ | $CH_3$ | F | CN | $-O-CH_2-CH=CH_2$ |
| $CH_3$ | $CH_3$ | F | CN | $-O-CH-COOC_2H_5$<br>\|<br>$CH_3$ |
| $CH_3$ | $CH_3$ | F | CN | $-O-CH_2-CH_2-OCH_3$ |
| $CH_3$ | $CH_3$ | F | CN | $-O-CH_2-C\equiv CH$ |
| $CH_3$ | $CH_3$ | F | CN | $CH_2F$<br>$-O-CH\langle$<br>$CH_2F$ |
| $CH_3$ | $CH_3$ | F | CN | $CH_3$<br>$-O-CH\langle$<br>$CH_3$ |

| $R^1$ | $R^2$ | $R^3$ | $R^4$ | $R^5$ |
|---|---|---|---|---|
| $CH_3$ | $CH_3$ | F | CN | $-O-CH-CN$<br>\|<br>$CH_3$ |
| $CH_3$ | $CH_3$ | F | CN | $-O-CH-COOCH_3$<br>\|<br>$CH_3$ |
| $CH_3$ | $CH_3$ | F | CN | $-O-CH_2-CH=CH-Cl$ |
| $CH_3$ | $CH_3$ | F | CN | $-O-CH_2-COO-(CH_2)_3-CH_3$ |
| $CH_3$ | $CH_3$ | F | CN | $-O-CH_2-COO-CH_2-CH_2-OC_2H_5$ |
| $CH_3$ | $CH_3$ | F | CN | $-O-(CH_2-CH_2-O)_2-C_2H_5$ |
| $CH_3$ | $CH_3$ | F | CN | $-O-CH_2-CH_2-O-(CH_2)_3-CH_3$ |
| $CH_3$ | $CH_3$ | F | CN | $-O-CH_2-CH=CH-CH_3$ |
| $CH_3$ | $CH_3$ | F | CN | $-O-(CH_2-CH_2-O)_2-CH_3$ |
| $CH_3$ | $CH_3$ | F | CN | $-S-CH_2-COOCH_3$ |
| $CH_3$ | $CH_3$ | F | CN | $-S-CH_2-COOC_2H_5$ |
| $CH_3$ | $CH_3$ | F | CN | $-S-CH_2-COO$ 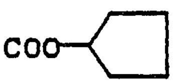 |
| $CH_3$ | $CH_3$ | F | CN | $-S-CH-COOC_2H_5$<br>\|<br>$CH_3$ |
| $CH_3$ | $CH_3$ | F | CN | $-S-CH-COO$ 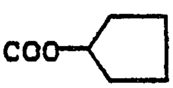<br>\|<br>$CH_3$ |
| $CH_3$ | $CH_3$ | F | CN | $-S-CH_2-CH=CH_2$ |
| $CH_3$ | $CH_3$ | F | CN | $-S-CH_2-COO-CH_2$ 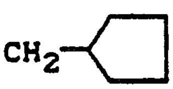 |

| $R^1$ | $R^2$ | $R^3$ | $R^4$ | $R^5$ |
|---|---|---|---|---|
| $CH_3$ | $CH_3$ | F | CN | $-S-CH_2-COO-CH_2-CH_2-OC_2H_5$ |
| $CH_3$ | $CH_3$ | F | CN | $-S-CH_2-COO-CH_2-CH_2-OCH_3$ |
| $CH_3$ | $CH_3$ | F | CN | $-S-CH_2-CH=CH_2$ |
| $CH_3$ | $CH_3$ | F | CN | $-S-CH_2-C\equiv CH$ |
| $CH_3$ | $CH_3$ | F | CN | $-S-CH(CH_3)_2$ |
| $CH_3$ | $CH_3$ | F | CN | 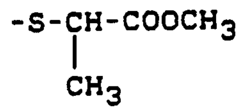 |
| $CH_3$ | $CH_3$ | F | CN | $-S-CH_2-COO-(CH_2)_3-CH_3$ |
| $CH_3$ | $CH_3$ | H | $CF_3$ | $-O-CH_2-CH_2-OC_2H_5$ |
| $CH_3$ | $CH_3$ | H | $CF_3$ | $-O-CH_2-CH=CH_2$ |
| $CH_3$ | $CH_3$ | H | $CF_3$ | 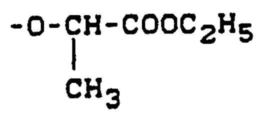 |
| $CH_3$ | $CH_3$ | H | $CF_3$ | $-O-CH_2-CH_2-OCH_3$ |
| $CH_3$ | $CH_3$ | H | $CF_3$ | $-O-CH_2-C\equiv CH$ |
| $CH_3$ | $CH_3$ | H | $CF_3$ | 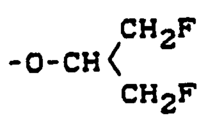 |
| $CH_3$ | $CH_3$ | H | $CF_3$ | 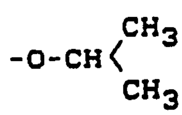 |
| $CH_3$ | $CH_3$ | H | $CF_3$ | 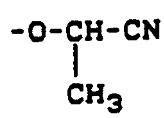 |
| $CH_3$ | $CH_3$ | H | $CF_3$ | 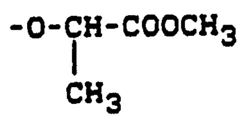 |

| $R^1$ | $R^2$ | $R^3$ | $R^4$ | $R^5$ |
|---|---|---|---|---|
| $CH_3$ | $CH_3$ | H | $CF_3$ | $-O-CH_2-CH=CH-Cl$ |
| $CH_3$ | $CH_3$ | H | $CF_3$ | $-O-CH_2-COO-(CH_2)_3-CH_3$ |
| $CH_3$ | $CH_3$ | H | $CF_3$ | $-O-CH_2-COO-CH_2-CH_2-OC_2H_5$ |
| $CH_3$ | $CH_3$ | H | $CF_3$ | $-O-(CH_2-CH_2-O)_2-C_2H_5$ |
| $CH_3$ | $CH_3$ | H | $CF_3$ | $-O-CH_2-CH_2-O-(CH_2)_3-CH_3$ |
| $CH_3$ | $CH_3$ | H | $CF_3$ | $-O-CH_2-CH=CH-CH_3$ |
| $CH_3$ | $CH_3$ | H | $CF_3$ | $-O-(CH_2-CH_2-O)_2-CH_3$ |
| $CH_3$ | $CH_3$ | H | $CF_3$ | $-S-CH_2-COOCH_3$ |
| $CH_3$ | $CH_3$ | H | $CF_3$ | $-S-CH_2-COOC_2H_5$ |
| $CH_3$ | $CH_3$ | H | $CF_3$ | $-S-CH_2-COO$ 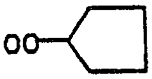 |
| $CH_3$ | $CH_3$ | H | $CF_3$ | $-S-CH(CH_3)-COOC_2H_5$ |
| $CH_3$ | $CH_3$ | H | $CF_3$ | $-S-CH(CH_3)-COO$ 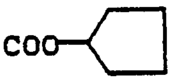 |
| $CH_3$ | $CH_3$ | H | $CF_3$ | $-S-CH_2-CH=CH_2$ |
| $CH_3$ | $CH_3$ | H | $CF_3$ | $-S-CH_2-COO-CH_2$ 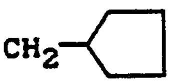 |
| $CH_3$ | $CH_3$ | H | $CF_3$ | $-S-CH_2-COO-CH_2-CH_2-OC_2H_5$ |
| $CH_3$ | $CH_3$ | H | $CF_3$ | $-S-CH_2-COO-CH_2-CH_2-OCH_3$ |
| $CH_3$ | $CH_3$ | H | $CF_3$ | $-S-CH_2-CH=CH_2$ |
| $CH_3$ | $CH_3$ | H | $CF_3$ | $-S-CH_2-C\equiv CH$ |

| $R^1$ | $R^2$ | $R^3$ | $R^4$ | $R^5$ |
|---|---|---|---|---|
| $CH_3$ | $CH_3$ | H | $CF_3$ | $-S-CH(CH_3)_2$ |
| $CH_3$ | $CH_3$ | H | $CF_3$ | $-S-CH-COOCH_3$<br>\|<br>$CH_3$ |
| $CH_3$ | $CH_3$ | H | $CF_3$ | $-S-CH_2-COO-(CH_2)_3-CH_3$ |
| $CH_3$ | $CH_3$ | F | $CF_3$ | $-O-CH_2-CH_2-OC_2H_5$ |
| $CH_3$ | $CH_3$ | F | $CF_3$ | $-O-CH_2-CH=CH_2$ |
| $CH_3$ | $CH_3$ | F | $CF_3$ | $-O-CH-COOC_2H_5$<br>\|<br>$CH_3$ |
| $CH_3$ | $CH_3$ | F | $CF_3$ | $-O-CH_2-CH_2-OCH_3$ |
| $CH_3$ | $CH_3$ | F | $CF_3$ | $-O-CH_2-C\equiv CH$ |
| $CH_3$ | $CH_3$ | F | $CF_3$ | $CH_2F$<br>$-O-CH\langle$<br>$CH_2F$ |
| $CH_3$ | $CH_3$ | F | $CF_3$ | $CH_3$<br>$-O-CH\langle$<br>$CH_3$ |
| $CH_3$ | $CH_3$ | F | $CF_3$ | $-O-CH-CN$<br>\|<br>$CH_3$ |
| $CH_3$ | $CH_3$ | F | $CF_3$ | $-O-CH-COOCH_3$<br>\|<br>$CH_3$ |
| $CH_3$ | $CH_3$ | F | $CF_3$ | $-O-CH_2-CH=CH-Cl$ |
| $CH_3$ | $CH_3$ | F | $CF_3$ | $-O-CH_2-COO-(CH_2)_3-CH_3$ |
| $CH_3$ | $CH_3$ | F | $CF_3$ | $-O-CH_2-COO-CH_2-CH_2-OC_2H_5$ |
| $CH_3$ | $CH_3$ | F | $CF_3$ | $-O-(CH_2-CH_2-O)_2-C_2H_5$ |

| $R^1$ | $R^2$ | $R^3$ | $R^4$ | $R^5$ |
|---|---|---|---|---|
| $CH_3$ | $CH_3$ | F | $CF_3$ | $-O-CH_2-CH_2-O-(CH_2)_3-CH_3$ |
| $CH_3$ | $CH_3$ | F | $CF_3$ | $-O-CH_2-CH=CH-CH_3$ |
| $CH_3$ | $CH_3$ | F | $CF_3$ | $-O-(CH_2-CH_2-O)_2-CH_3$ |
| $CH_3$ | $CH_3$ | F | $CF_3$ | $-S-CH_2-COOCH_3$ |
| $CH_3$ | $CH_3$ | F | $CF_3$ | $-S-CH_2-COOC_2H_5$ |
| $CH_3$ | $CH_3$ | F | $CF_3$ | $-S-CH_2-COO-$ 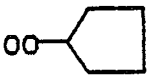 |
| $CH_3$ | $CH_3$ | F | $CF_3$ | $-S-CH-COOC_2H_5$<br>$CH_3$ |
| $CH_3$ | $CH_3$ | F | $CF_3$ | $-S-CH-COO-$ 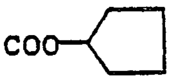<br>$CH_3$ |
| $CH_3$ | $CH_3$ | F | $CF_3$ | $-S-CH_2-CH=CH_2$ |
| $CH_3$ | $CH_3$ | F | $CF_3$ | $-S-CH_2-COO-CH_2-$ 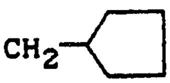 |
| $CH_3$ | $CH_3$ | F | $CF_3$ | $-S-CH_2-COO-CH_2-CH_2-OC_2H_5$ |
| $CH_3$ | $CH_3$ | F | $CF_3$ | $-S-CH_2-COO-CH_2-CH_2-OCH_3$ |
| $CH_3$ | $CH_3$ | F | $CF_3$ | $-S-CH_2-CH=CH_2$ |
| $CH_3$ | $CH_3$ | F | $CF_3$ | $-S-CH_2-C\equiv CH$ |
| $CH_3$ | $CH_3$ | F | $CF_3$ | $-S-CH(CH_3)_2$ |
| $CH_3$ | $CH_3$ | F | $CF_3$ | $-S-CH-COOCH_3$<br>$CH_3$ |

| $R^1$ | $R^2$ | $R^3$ | $R^4$ | $R^5$ |
|---|---|---|---|---|
| $CH_3$ | $CH_3$ | F | $CF_3$ | $-S-CH_2-COO-(CH_2)_3-CH_3$ |
| $CH_3$ | $CH_3$ | F | $OCH_3$ | $-O-CH_2-CH_2-OC_2H_5$ |
| $CH_3$ | $CH_3$ | F | $OCH_3$ | $-O-CH_2-CH=CH_2$ |
| $CH_3$ | $CH_3$ | F | $OCH_3$ | $-O-\underset{\mid \atop CH_3}{CH}-COOC_2H_5$ |
| $CH_3$ | $CH_3$ | F | $OCH_3$ | $-O-CH_2-CH_2-OCH_3$ |
| $CH_3$ | $CH_3$ | F | $OCH_3$ | $-O-CH_2-C\equiv CH$ |
| $CH_3$ | $CH_3$ | F | $OCH_3$ | $-O-CH\left\langle \begin{matrix} CH_2F \\ CH_2F \end{matrix} \right.$ |
| $CH_3$ | $CH_3$ | F | $OCH_3$ | $-O-CH\left\langle \begin{matrix} CH_3 \\ CH_3 \end{matrix} \right.$ |
| $CH_3$ | $CH_3$ | F | $OCH_3$ | $-O-\underset{\mid \atop CH_3}{CH}-CN$ |
| $CH_3$ | $CH_3$ | F | $OCH_3$ | $-O-\underset{\mid \atop CH_3}{CH}-COOCH_3$ |
| $CH_3$ | $CH_3$ | F | $OCH_3$ | $-O-CH_2-CH=CH-Cl$ |
| $CH_3$ | $CH_3$ | F | $OCH_3$ | $-O-CH_2-COO-(CH_2)_3-CH_3$ |
| $CH_3$ | $CH_3$ | F | $OCH_3$ | $-O-CH_2-COO-CH_2-CH_2-OC_2H_5$ |
| $CH_3$ | $CH_3$ | F | $OCH_3$ | $-O-(CH_2-CH_2-O)_2-C_2H_5$ |
| $CH_3$ | $CH_3$ | F | $OCH_3$ | $-O-CH_2-CH_2-O-(CH_2)_3-CH_3$ |
| $CH_3$ | $CH_3$ | F | $OCH_3$ | $-O-CH_2-CH=CH-CH_3$ |
| $CH_3$ | $CH_3$ | F | $OCH_3$ | $-O-(CH_2-CH_2-O)_2-CH_3$ |

| $R^1$ | $R^2$ | $R^3$ | $R^4$ | $R^5$ |
|---|---|---|---|---|
| $CH_3$ | $CH_3$ | F | $OCH_3$ | $-S-CH_2-COOCH_3$ |
| $CH_3$ | $CH_3$ | F | $OCH_3$ | $-S-CH_2-COOC_2H_5$ |
| $CH_3$ | $CH_3$ | F | $OCH_3$ | $-S-CH_2-COO$ 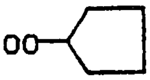 |
| $CH_3$ | $CH_3$ | F | $OCH_3$ | $-S-CH-COOC_2H_5$<br>$CH_3$ |
| $CH_3$ | $CH_3$ | F | $OCH_3$ | $-S-CH-COO$ 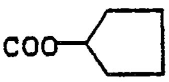<br>$CH_3$ |
| $CH_3$ | $CH_3$ | F | $OCH_3$ | $-S-CH_2-CH=CH_2$ |
| $CH_3$ | $CH_3$ | F | $OCH_3$ | $-S-CH_2-COO-CH_2$ 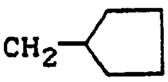 |
| $CH_3$ | $CH_3$ | F | $OCH_3$ | $-S-CH_2-COO-CH_2-CH_2-OC_2H_5$ |
| $CH_3$ | $CH_3$ | F | $OCH_3$ | $-S-CH_2-COO-CH_2-CH_2-OCH_3$ |
| $CH_3$ | $CH_3$ | F | $OCH_3$ | $-S-CH_2-CH=CH_2$ |
| $CH_3$ | $CH_3$ | F | $OCH_3$ | $-S-CH_2-C{\equiv}CH$ |
| $CH_3$ | $CH_3$ | F | $OCH_3$ | $-S-CH(CH_3)_2$ |
| $CH_3$ | $CH_3$ | F | $OCH_3$ | $-S-CH-COOCH_3$<br>$CH_3$ |
| $CH_3$ | $CH_3$ | F | $OCH_3$ | $-S-CH_2-COO-(CH_2)_3-CH_3$ |
| $CH_3$ | $CH_3$ | F | $NO_2$ | $-O-CH_2-CH_2-OC_2H_5$ |
| $CH_3$ | $CH_3$ | F | $NO_2$ | $-O-CH_2-CH=CH_2$ |

| $R^1$ | $R^2$ | $R^3$ | $R^4$ | $R^5$ |
|---|---|---|---|---|
| $CH_3$ | $CH_3$ | F | $NO_2$ | $-O-CH-COOC_2H_5$<br>\|<br>$CH_3$ |
| $CH_3$ | $CH_3$ | F | $NO_2$ | $-O-CH_2-CH_2-OCH_3$ |
| $CH_3$ | $CH_3$ | F | $NO_2$ | $-O-CH_2-C\equiv CH$ |
| $CH_3$ | $CH_3$ | F | $NO_2$ | $CH_2F$<br>$-O-CH\langle$<br>$CH_2F$ |
| $CH_3$ | $CH_3$ | F | $NO_2$ | $CH_3$<br>$-O-CH\langle$<br>$CH_3$ |
| $CH_3$ | $CH_3$ | F | $NO_2$ | $-O-CH-CN$<br>\|<br>$CH_3$ |
| $CH_3$ | $CH_3$ | F | $NO_2$ | $-O-CH-COOCH_3$<br>\|<br>$CH_3$ |
| $CH_3$ | $CH_3$ | F | $NO_2$ | $-O-CH_2-CH=CH-Cl$ |
| $CH_3$ | $CH_3$ | F | $NO_2$ | $-O-CH_2-COO-(CH_2)_3-CH_3$ |
| $CH_3$ | $CH_3$ | F | $NO_2$ | $-O-CH_2-COO-CH_2-CH_2-OC_2H_5$ |
| $CH_3$ | $CH_3$ | F | $NO_2$ | $-O-(CH_2-CH_2-O)_2-C_2H_5$ |
| $CH_3$ | $CH_3$ | F | $NO_2$ | $-O-CH_2-CH_2-O-(CH_2)_3-CH_3$ |
| $CH_3$ | $CH_3$ | F | $NO_2$ | $-O-CH_2-CH=CH-CH_3$ |
| $CH_3$ | $CH_3$ | F | $NO_2$ | $-O-(CH_2-CH_2-O)_2-CH_3$ |
| $CH_3$ | $CH_3$ | F | $NO_2$ | $-S-CH_2-COOCH_3$ |
| $CH_3$ | $CH_3$ | F | $NO_2$ | $-S-CH_2-COOC_2H_5$ |

| $R^1$ | $R^2$ | $R^3$ | $R^4$ | $R^5$ |
|---|---|---|---|---|
| $CH_3$ | $CH_3$ | F | $NO_2$ | $-S-CH_2-COO$ 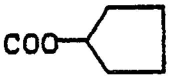 |
| $CH_3$ | $CH_3$ | F | $NO_2$ | $-S-CH-COOC_2H_5$<br>$CH_3$ |
| $CH_3$ | $CH_3$ | F | $NO_2$ | $-S-CH-COO$ 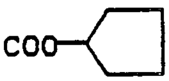<br>$CH_3$ |
| $CH_3$ | $CH_3$ | F | $NO_2$ | $-S-CH_2-CH=CH_2$ |
| $CH_3$ | $CH_3$ | F | $NO_2$ | $-S-CH_2-COO-CH_2$ 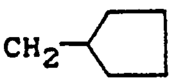 |
| $CH_3$ | $CH_3$ | F | $NO_2$ | $-S-CH_2-COO-CH_2-CH_2-OC_2H_5$ |
| $CH_3$ | $CH_3$ | F | $NO_2$ | $-S-CH_2-COO-CH_2-CH_2-OCH_3$ |
| $CH_3$ | $CH_3$ | F | $NO_2$ | $-S-CH_2-CH=CH_2$ |
| $CH_3$ | $CH_3$ | F | $NO_2$ | $-S-CH_2-C\equiv CH$ |
| $CH_3$ | $CH_3$ | F | $NO_2$ | $-S-CH(CH_3)_2$ |
| $CH_3$ | $CH_3$ | F | $NO_2$ | $-S-CH-COOCH_3$<br>$CH_3$ |
| $CH_3$ | $CH_3$ | F | $NO_2$ | $-S-CH_2-COO-(CH_2)_3-CH_3$ |
| $CH_3$ | $CH_3$ | H | $NO_2$ | $-O-CH_2-CH_2-OC_2H_5$ |
| $CH_3$ | $CH_3$ | H | $NO_2$ | $-O-CH_2-CH=CH_2$ |
| $CH_3$ | $CH_3$ | H | $NO_2$ | $-O-CH-COOC_2H_5$<br>$CH_3$ |

| $R^1$ | $R^2$ | $R^3$ | $R^4$ | $R^5$ |
|---|---|---|---|---|
| $CH_3$ | $CH_3$ | H | $NO_2$ | $-O-CH_2-CH_2-OCH_3$ |
| $CH_3$ | $CH_3$ | H | $NO_2$ | $-O-CH_2-C\equiv CH$ |
| $CH_3$ | $CH_3$ | H | $NO_2$ | $-O-CH\left<\begin{matrix}CH_2F\\CH_2F\end{matrix}\right.$ |
| $CH_3$ | $CH_3$ | H | $NO_2$ | $-O-CH\left<\begin{matrix}CH_3\\CH_3\end{matrix}\right.$ |
| $CH_3$ | $CH_3$ | H | $NO_2$ | $-O-\underset{CH_3}{\underset{\vert}{C}}H-CN$ |
| $CH_3$ | $CH_3$ | H | $NO_2$ | $-O-\underset{CH_3}{\underset{\vert}{C}}H-COOCH_3$ |
| $CH_3$ | $CH_3$ | H | $NO_2$ | $-O-CH_2-CH=CH-Cl$ |
| $CH_3$ | $CH_3$ | H | $NO_2$ | $-O-CH_2-COO-(CH_2)_3-CH_3$ |
| $CH_3$ | $CH_3$ | H | $NO_2$ | $-O-CH_2-COO-CH_2-CH_2-OC_2H_5$ |
| $CH_3$ | $CH_3$ | H | $NO_2$ | $-O-(CH_2-CH_2-O)_2-C_2H_5$ |
| $CH_3$ | $CH_3$ | H | $NO_2$ | $-O-CH_2-CH_2-O-(CH_2)_3-CH_3$ |
| $CH_3$ | $CH_3$ | H | $NO_2$ | $-O-CH_2-CH=CH-CH_3$ |
| $CH_3$ | $CH_3$ | H | $NO_2$ | $-O-(CH_2-CH_2-O)_2-CH_3$ |
| $CH_3$ | $CH_3$ | H | $NO_2$ | $-S-CH_2-COOCH_3$ |
| $CH_3$ | $CH_3$ | H | $NO_2$ | $-S-CH_2-COOC_2H_5$ |
| $CH_3$ | $CH_3$ | H | $NO_2$ | $-S-CH_2-COO$ 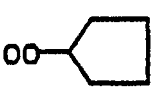 |

| $R^1$ | $R^2$ | $R^3$ | $R^4$ | $R^5$ |
|---|---|---|---|---|
| $CH_3$ | $CH_3$ | H | $NO_2$ | $-S-CH-COOC_2H_5$<br>\|<br>$CH_3$ |
| $CH_3$ | $CH_3$ | H | $NO_2$ | 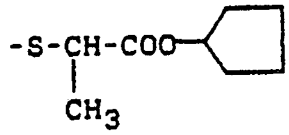 |
| $CH_3$ | $CH_3$ | H | $NO_2$ | $-S-CH_2-CH=CH_2$ |
| $CH_3$ | $CH_3$ | H | $NO_2$ | 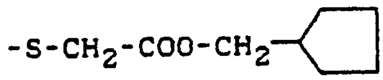 |
| $CH_3$ | $CH_3$ | H | $NO_2$ | $-S-CH_2-COO-CH_2-CH_2-OC_2H_5$ |
| $CH_3$ | $CH_3$ | H | $NO_2$ | $-S-CH_2-COO-CH_2-CH_2-OCH_3$ |
| $CH_3$ | $CH_3$ | H | $NO_2$ | $-S-CH_2-CH=CH_2$ |
| $CH_3$ | $CH_3$ | H | $NO_2$ | $-S-CH_2-C\equiv CH$ |
| $CH_3$ | $CH_3$ | H | $NO_2$ | $-S-CH(CH_3)_2$ |
| $CH_3$ | $CH_3$ | H | $NO_2$ | $-S-CH-COOCH_3$<br>\|<br>$CH_3$ |
| $CH_3$ | $CH_3$ | H | $NO_2$ | $-S-CH_2-COO-(CH_2)_3-CH_3$ |
| $CH_3$ | $CH_3$ | H | CN | $-O-CH_2-CH_2-OC_2H_5$ |
| $CH_3$ | $CH_3$ | H | CN | $-O-CH_2-CH=CH_2$ |
| $CH_3$ | $CH_3$ | H | CN | $-O-CH-COOC_2H_5$<br>\|<br>$CH_3$ |
| $CH_3$ | $CH_3$ | H | CN | $-O-CH_2-CH_2-OCH_3$ |
| $CH_3$ | $CH_3$ | H | CN | $-O-CH_2-C\equiv CH$ |

| $R^1$ | $R^2$ | $R^3$ | $R^4$ | $R^5$ |
|---|---|---|---|---|
| $CH_3$ | $CH_3$ | H | CN | 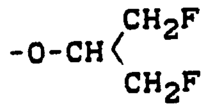 |
| $CH_3$ | $CH_3$ | H | CN | 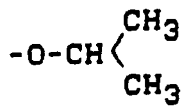 |
| $CH_3$ | $CH_3$ | H | CN | 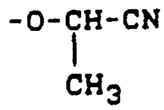 |
| $CH_3$ | $CH_3$ | H | CN | 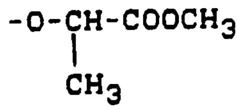 |
| $CH_3$ | $CH_3$ | H | CN | $-O-CH_2-CH=CH-Cl$ |
| $CH_3$ | $CH_3$ | H | CN | $-O-CH_2-COO-(CH_2)_3-CH_3$ |
| $CH_3$ | $CH_3$ | H | CN | $-O-CH_2-COO-CH_2-CH_2-OC_2H_5$ |
| $CH_3$ | $CH_3$ | H | CN | $-O-(CH_2-CH_2-O)_2-C_2H_5$ |
| $CH_3$ | $CH_3$ | H | CN | $-O-CH_2-CH_2-O-(CH_2)_3-CH_3$ |
| $CH_3$ | $CH_3$ | H | CN | $-O-CH_2-CH=CH-CH_3$ |
| $CH_3$ | $CH_3$ | H | CN | $-O-(CH_2-CH_2-O)_2-CH_3$ |
| $CH_3$ | $CH_3$ | H | CN | $-S-CH_2-COOCH_3$ |
| $CH_3$ | $CH_3$ | H | CN | $-S-CH_2-COOC_2H_5$ |
| $CH_3$ | $CH_3$ | H | CN | $-S-CH_2-COO$ 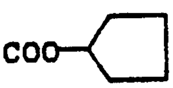 |
| $CH_3$ | $CH_3$ | H | CN | 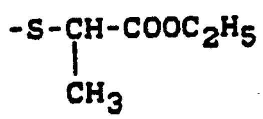 |

| $R^1$ | $R^2$ | $R^3$ | $R^4$ | $R^5$ |
|---|---|---|---|---|
| $CH_3$ | $CH_3$ | H | CN | $-S-CH-COO-$ 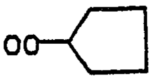<br>\|<br>$CH_3$ |
| $CH_3$ | $CH_3$ | H | CN | $-S-CH_2-CH=CH_2$ |
| $CH_3$ | $CH_3$ | H | CN | $-S-CH_2-COO-CH_2-$ 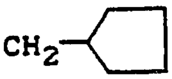 |
| $CH_3$ | $CH_3$ | H | CN | $-S-CH_2-COO-CH_2-CH_2-OC_2H_5$ |
| $CH_3$ | $CH_3$ | H | CN | $-S-CH_2-COO-CH_2-CH_2-OCH_3$ |
| $CH_3$ | $CH_3$ | H | CN | $-S-CH_2-CH=CH_2$ |
| $CH_3$ | $CH_3$ | H | CN | $-S-CH_2-C{\equiv}CH$ |
| $CH_3$ | $CH_3$ | H | CN | $-S-CH(CH_3)_2$ |
| $CH_3$ | $CH_3$ | H | CN | $-S-CH-COOCH_3$<br>\|<br>$CH_3$ |
| $CH_3$ | $CH_3$ | H | CN | $-S-CH_2-COO-(CH_2)_3-CH_3$ |
| $CH_3$ | $CH_3$ | F | $CH_3$ | $-O-CH_2-CH_2-OC_2H_5$ |
| $CH_3$ | $CH_3$ | F | $CH_3$ | $-O-CH_2-CH=CH_2$ |
| $CH_3$ | $CH_3$ | F | $CH_3$ | $-O-CH-COOC_2H_5$<br>\|<br>$CH_3$ |
| $CH_3$ | $CH_3$ | F | $CH_3$ | $-O-CH_2-CH_2-OCH_3$ |
| $CH_3$ | $CH_3$ | F | $CH_3$ | $-O-CH_2-C{\equiv}CH$ |
| $CH_3$ | $CH_3$ | F | $CH_3$ | $-O-CH<$ $CH_2F$ / $CH_2F$ |

| $R^1$ | $R^2$ | $R^3$ | $R^4$ | $R^5$ |
|---|---|---|---|---|
| $CH_3$ | $CH_3$ | F | $CH_3$ | $-O-CH(CH_3)_2$ |
| $CH_3$ | $CH_3$ | F | $CH_3$ | $-O-CH(CH_3)-CN$ |
| $CH_3$ | $CH_3$ | F | $CH_3$ | $-O-CH(CH_3)-COOCH_3$ |
| $CH_3$ | $CH_3$ | F | $CH_3$ | $-O-CH_2-CH=CH-Cl$ |
| $CH_3$ | $CH_3$ | F | $CH_3$ | $-O-CH_2-COO-(CH_2)_3-CH_3$ |
| $CH_3$ | $CH_3$ | F | $CH_3$ | $-O-CH_2-COO-CH_2-CH_2-OC_2H_5$ |
| $CH_3$ | $CH_3$ | F | $CH_3$ | $-O-(CH_2-CH_2-O)_2-C_2H_5$ |
| $CH_3$ | $CH_3$ | F | $CH_3$ | $-O-CH_2-CH_2-O-(CH_2)_3-CH_3$ |
| $CH_3$ | $CH_3$ | F | $CH_3$ | $-O-CH_2-CH=CH-CH_3$ |
| $CH_3$ | $CH_3$ | F | $CH_3$ | $-O-(CH_2-CH_2-O)_2-CH_3$ |
| $CH_3$ | $CH_3$ | F | $CH_3$ | $-S-CH_2-COOCH_3$ |
| $CH_3$ | $CH_3$ | F | $CH_3$ | $-S-CH_2-COOC_2H_5$ |
| $CH_3$ | $CH_3$ | F | $CH_3$ | $-S-CH_2-COO-$ 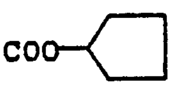 |
| $CH_3$ | $CH_3$ | F | $CH_3$ | $-S-CH(CH_3)-COOC_2H_5$ |
| $CH_3$ | $CH_3$ | F | $CH_3$ | $-S-CH(CH_3)-COO-$ 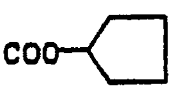 |

| $R^1$ | $R^2$ | $R^3$ | $R^4$ | $R^5$ |
|---|---|---|---|---|
| $CH_3$ | $CH_3$ | F | $CH_3$ | $-S-CH_2-CH=CH_2$ |
| $CH_3$ | $CH_3$ | F | $CH_3$ | $-S-CH_2-COO-CH_2-$ 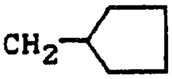 |
| $CH_3$ | $CH_3$ | F | $CH_3$ | $-S-CH_2-COO-CH_2-CH_2-OC_2H_5$ |
| $CH_3$ | $CH_3$ | F | $CH_3$ | $-S-CH_2-COO-CH_2-CH_2-OCH_3$ |
| $CH_3$ | $CH_3$ | F | $CH_3$ | $-S-CH_2-CH=CH_2$ |
| $CH_3$ | $CH_3$ | F | $CH_3$ | $-S-CH_2-C\equiv CH$ |
| $CH_3$ | $CH_3$ | F | $CH_3$ | $-S-CH(CH_3)_2$ |
| $CH_3$ | $CH_3$ | F | $CH_3$ | $-S-CH-COOCH_3$<br>$\vert$<br>$CH_3$ |
| $CH_3$ | $CH_3$ | F | $CH_3$ | $-S-CH_2-COO-(CH_2)_3-CH_3$ |
| $CH_3$ | $CH_3$ | H | $CH_3$ | $-O-CH_2-CH_2-OC_2H_5$ |
| $CH_3$ | $CH_3$ | H | $CH_3$ | $-O-CH_2-CH=CH_2$ |
| $CH_3$ | $CH_3$ | H | $CH_3$ | $-O-CH-COOC_2H_5$<br>$\vert$<br>$CH_3$ |
| $CH_3$ | $CH_3$ | H | $CH_3$ | $-O-CH_2-CH_2-OCH_3$ |
| $CH_3$ | $CH_3$ | H | $CH_3$ | $-O-CH_2-C\equiv CH$ |
| $CH_3$ | $CH_3$ | H | $CH_3$ | $CH_2F$<br>$-O-CH<$<br>$CH_2F$ |
| $CH_3$ | $CH_3$ | H | $CH_3$ | $CH_3$<br>$-O-CH<$<br>$CH_3$ |

| $R^1$ | $R^2$ | $R^3$ | $R^4$ | $R^5$ |
|---|---|---|---|---|
| $CH_3$ | $CH_3$ | H | $CH_3$ | $-O-\underset{CH_3}{\underset{\mid}{CH}}-CN$ |
| $CH_3$ | $CH_3$ | H | $CH_3$ | $-O-\underset{CH_3}{\underset{\mid}{CH}}-COOCH_3$ |
| $CH_3$ | $CH_3$ | H | $CH_3$ | $-O-CH_2-CH=CH-Cl$ |
| $CH_3$ | $CH_3$ | H | $CH_3$ | $-O-CH_2-COO-(CH_2)_3-CH_3$ |
| $CH_3$ | $CH_3$ | H | $CH_3$ | $-O-CH_2-COO-CH_2-CH_2-OC_2H_5$ |
| $CH_3$ | $CH_3$ | H | $CH_3$ | $-O-(CH_2-CH_2-O)_2-C_2H_5$ |
| $CH_3$ | $CH_3$ | H | $CH_3$ | $-O-CH_2-CH_2-O-(CH_2)_3-CH_3$ |
| $CH_3$ | $CH_3$ | H | $CH_3$ | $-O-CH_2-CH=CH-CH_3$ |
| $CH_3$ | $CH_3$ | H | $CH_3$ | $-O-(CH_2-CH_2-O)_2-CH_3$ |
| $CH_3$ | $CH_3$ | H | $CH_3$ | $-S-CH_2-COOCH_3$ |
| $CH_3$ | $CH_3$ | H | $CH_3$ | $-S-CH_2-COOC_2H_5$ |
| $CH_3$ | $CH_3$ | H | $CH_3$ | $-S-CH_2-COO$ 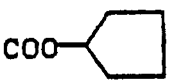 |
| $CH_3$ | $CH_3$ | H | $CH_3$ | $-S-\underset{CH_3}{\underset{\mid}{CH}}-COOC_2H_5$ |
| $CH_3$ | $CH_3$ | H | $CH_3$ | $-S-\underset{CH_3}{\underset{\mid}{CH}}-COO$ 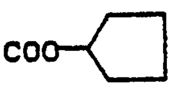 |
| $CH_3$ | $CH_3$ | H | $CH_3$ | $-S-CH_2-CH=CH_2$ |
| $CH_3$ | $CH_3$ | H | $CH_3$ | $-S-CH_2-COO-CH_2$ 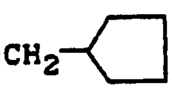 |

| $R^1$ | $R^2$ | $R^3$ | $R^4$ | $R^5$ |
|---|---|---|---|---|
| $CH_3$ | $CH_3$ | H | $CH_3$ | $-S-CH_2-COO-CH_2-CH_2-OC_2H_5$ |
| $CH_3$ | $CH_3$ | H | $CH_3$ | $-S-CH_2-COO-CH_2-CH_2-OCH_3$ |
| $CH_3$ | $CH_3$ | H | $CH_3$ | $-S-CH_2-CH=CH_2$ |
| $CH_3$ | $CH_3$ | H | $CH_3$ | $-S-CH_2-C\equiv CH$ |
| $CH_3$ | $CH_3$ | H | $CH_3$ | $-S-CH(CH_3)_2$ |
| $CH_3$ | $CH_3$ | H | $CH_3$ | $-S-CH-COOCH_3$<br>\|<br>$CH_3$ |
| $CH_3$ | $CH_3$ | H | $CH_3$ | $-S-CH_2-COO-(CH_2)_3-CH_3$ |
| $CH_3$ | $CH_3$ | F | $OCH_3$ | $-O-CH_2-CH_2-OC_2H_5$ |
| $CH_3$ | $CH_3$ | F | $OCH_3$ | $-O-CH_2-CH=CH_2$ |
| $CH_3$ | $CH_3$ | F | $OCH_3$ | $-O-CH-COOC_2H_5$<br>\|<br>$CH_3$ |
| $CH_3$ | $CH_3$ | F | $OCH_3$ | $-O-CH_2-CH_2-OCH_3$ |
| $CH_3$ | $CH_3$ | F | $OCH_3$ | $-O-CH_2-C\equiv CH$ |
| $CH_3$ | $CH_3$ | F | $OCH_3$ | $CH_2F$<br>$-O-CH\langle$<br>$CH_2F$ |
| $CH_3$ | $CH_3$ | F | $OCH_3$ | $CH_3$<br>$-O-CH\langle$<br>$CH_3$ |
| $CH_3$ | $CH_3$ | F | $OCH_3$ | $-O-CH-CN$<br>\|<br>$CH_3$ |
| $CH_3$ | $CH_3$ | F | $OCH_3$ | $-O-CH-COOCH_3$<br>\|<br>$CH_3$ |

| $R^1$ | $R^2$ | $R^3$ | $R^4$ | $R^5$ |
|---|---|---|---|---|
| $CH_3$ | $CH_3$ | F | $OCH_3$ | $-O-CH_2-CH=CH-Cl$ |
| $CH_3$ | $CH_3$ | F | $OCH_3$ | $-O-CH_2-COO-(CH_2)_3-CH_3$ |
| $CH_3$ | $CH_3$ | F | $OCH_3$ | $-O-CH_2-COO-CH_2-CH_2-OC_2H_5$ |
| $CH_3$ | $CH_3$ | F | $OCH_3$ | $-O-(CH_2-CH_2-O)_2-C_2H_5$ |
| $CH_3$ | $CH_3$ | F | $OCH_3$ | $-O-CH_2-CH_2-O-(CH_2)_3-CH_3$ |
| $CH_3$ | $CH_3$ | F | $OCH_3$ | $-O-CH_2-CH=CH-CH_3$ |
| $CH_3$ | $CH_3$ | F | $OCF_3$ | $-O-(CH_2-CH_2-O)_2-CH_3$ |
| $CH_3$ | $CH_3$ | F | $OCF_3$ | $-O-CH_2-CH=CH-CH_3$ |
| $CH_3$ | $CH_3$ | F | $OCF_3$ | $-O-(CH_2-CH_2-O)_2-CH_3$ |
| $CH_3$ | $CH_3$ | F | $OCF_3$ | $-S-CH_2-COOC_2H_5$ |
| $CH_3$ | $CH_3$ | F | $OCF_3$ | $-S-CH_2-COO-CH_2-CH_2-OCH_3$ |
| $CH_3$ | $CH_3$ | F | $OCF_3$ | $-O-CH_2-CH_2-OC_2H_5$ |
| $CH_3$ | $CH_3$ | F | $OCF_3$ | $-O-CH_2-CH=CH_2$ |
| $CH_3$ | $CH_3$ | F | $OCF_3$ | $-O-CH_2-C\equiv CH$ |
| $CF_3$ | $CF_3$ | F | CN | $-O-CH_2-CH_2-O-C_2H_5$ |
| $CF_3$ | $CF_3$ | F | CN | $-O-CH_2-CH=CH_2$ |
| $CF_3$ | $CF_3$ | F | CN | $-O-CH_2-C\equiv CH$ |
| $CF_3$ | $CF_3$ | F | CN | $-S-CH_2-COOC_2H_5$ |
| $CF_3$ | $CF_3$ | F | CN | $-S-CH_2-COO-CH_2CH_2-OCH_3$ |
| $CF_3$ | $CF_3$ | F | CN | $-O-(CH_2-CH_2-O)_2-CH_3$ |
| $CF_3$ | $CF_3$ | F | CN | $-O-CH_2-CH=CH-CH_3$ |
| $CF_3$ | $CF_3$ | F | CN | $-O-CH_2-CH_2-O-(CH_2)_3-CH_3$ |

| $R^1$ | $R^2$ | $R^3$ | $R^4$ | $R^5$ |
|---|---|---|---|---|
| $CF_3$ | $CF_3$ | F | CN | $-S-CH-COOC_2H_5$<br>|<br>$CH_3$ |
| $CH_3$ | H | F | CN | $-O-CH_2-CH_2-O-(CH_2)_3-CH_3$ |
| $CH_3$ | H | F | CN | $-O-CH_2-CH=CH-CH_3$ |
| $CH_3$ | H | F | CN | $-O-(CH_2-CH_2-O)_2-CH_3$ |
| $CH_3$ | H | F | CN | $-S-CH_2-COOC_2H_5$ |
| $CH_3$ | H | F | CN | $-S-CH_2-COO-CH_2-CH_2-OCH_3$ |
| $CH_3$ | H | F | CN | $-O-CH_2-CH_2-OC_2H_5$ |
| $CH_3$ | H | F | CN | $-O-CH_2-CH=CH_2$ |
| $CH_3$ | H | F | CN | $-O-CH_2-C{\equiv}CH$ |
| H | $CH_3$ | F | CN | $-O-CH_2-CH_2-O-C_2H_5$ |
| H | $CH_3$ | F | CN | $-O-CH_2-CH_2-O-C_2H_5$ |
| H | $CH_3$ | F | CN | $-O-CH_2-CH=CH_2$ |
| H | $CH_3$ | F | CN | $-O-CH_2-C{\equiv}CH$ |
| H | $CH_3$ | F | CN | $-S-CH_2-COOC_2H_5$ |
| H | $CH_3$ | F | CN | $-S-CH_2-COO-CH_2CH_2-OCH_3$ |
| H | $CH_3$ | F | CN | $-O-(CH_2-CH_2-O)_2-CH_3$ |
| H | $CH_3$ | F | CN | $-O-CH_2-CH=CH-CH_3$ |
| H | $CH_3$ | F | CN | $-O-CH_2-CH_2-O-(CH_2)_3-CH_3$ |
| H | $CH_3$ | F | CN | $-S-CH-COOC_2H_5$<br>|<br>$CH_3$ |

| $R^1$ | $R^2$ | $R^3$ | $R^4$ | $R^5$ |
|---|---|---|---|---|
| $C_2H_5$ | $CH_3$ | F | CN | $-O-CH_2-CH_2-O-(CH_2)_3-CH_3$ |
| $C_2H_5$ | $CH_3$ | F | CN | $-O-CH_2-CH=CH-CH_3$ |
| $C_2H_5$ | $CH_3$ | F | CN | $-O-(CH_2-CH_2-O)_2-CH_3$ |
| $C_2H_5$ | $CH_3$ | F | CN | $-S-CH_2-COOC_2H_5$ |
| $C_2H_5$ | $CH_3$ | F | CN | $-S-CH_2-COO-CH_2-CH_2-OCH_3$ |
| $C_2H_5$ | $CH_3$ | F | CN | $-O-CH_2-CH_2-OC_2H_5$ |
| $C_2H_5$ | $CH_3$ | F | CN | $-O-CH_2-CH=CH_2$ |
| $C_2H_5$ | $CH_3$ | F | CN | $-O-CH_2-C\equiv CH$ |
| $CH_3$ | $C_2H_5$ | F | CN | $-O-CH_2-CH_2-O-C_2H_5$ |
| $CH_3$ | $C_2H_5$ | F | CN | $-O-CH_2-CH=CH_2$ |
| $CH_3$ | $C_2H_5$ | F | CN | $-O-CH_2-C\equiv CH$ |
| $CH_3$ | $C_2H_5$ | F | CN | $-S-CH_2-COOC_2H_5$ |
| $CH_3$ | $C_2H_5$ | F | CN | $-S-CH_2-COO-CH_2CH_2-OCH_3$ |
| $CH_3$ | $C_2H_5$ | F | CN | $-O-(CH_2-CH_2-O)_2-CH_3$ |
| $CH_3$ | $C_2H_5$ | F | CN | $-O-CH_2-CH=CH-CH_3$ |
| $CH_3$ | $C_2H_5$ | F | CN | $-O-CH_2-CH_2-O-(CH_2)_3-CH_3$ |
| $CH_3$ | $C_2H_5$ | F | CN | $-S-CH(CH_3)-COOC_2H_5$ |
| $CF_3$ | $CH_3$ | F | CN | $-O-CH_2-CH_2-O-(CH_2)_3-CH_3$ |
| $CF_3$ | $CH_3$ | F | CN | $-O-CH_2-CH=CH-CH_3$ |
| $CF_3$ | $CH_3$ | F | CN | $-O-(CH_2-CH_2-O)_2-CH_3$ |

| $R^1$ | $R^2$ | $R^3$ | $R^4$ | $R^5$ |
|---|---|---|---|---|
| $CF_3$ | $CH_3$ | F | CN | $-S-CH_2-COOC_2H_5$ |
| $CF_3$ | $CH_3$ | F | CN | $-S-CH_2-COO-CH_2-CH_2-OCH_3$ |
| $CF_3$ | $CH_3$ | F | CN | $-O-CH_2-CH_2-OC_2H_5$ |
| $CF_3$ | $CH_3$ | F | CN | $-O-CH_2-CH=CH_2$ |
| $CF_3$ | $CH_3$ | F | CN | $-O-CH_2-C\equiv CH$ |
| $CH_3$ | $CF_3$ | F | CN | $-O-CH_2-CH_2-O-C_2H_5$ |
| $CH_3$ | $CF_3$ | F | CN | $-O-CH_2-CH_2-O-C_2H_5$ |
| $CH_3$ | $CF_3$ | F | CN | $-O-CH_2-CH=CH_2$ |
| $CH_3$ | $CF_3$ | F | CN | $-O-CH_2-C\equiv CH$ |
| $CH_3$ | $CF_3$ | F | CN | $-S-CH_2-COOC_2H_5$ |
| $CH_3$ | $CF_3$ | F | CN | $-S-CH_2-COO-CH_2CH_2-OCH_3$ |
| $CH_3$ | $CF_3$ | F | CN | $-O-(CH_2-CH_2-O)_2-CH_3$ |
| $CH_3$ | $CF_3$ | F | CN | $-O-CH_2-CH=CH-CH_3$ |
| $CH_3$ | $CF_3$ | F | CN | $-O-CH_2-CH_2-O-(CH_2)_3-CH_3$ |
| $CH_3$ | $CF_3$ | F | CN | $-S-CH(CH_3)-COOC_2H_5$ |
| $CH_3$ | $CH_3$ | F | $-OCHF_2$ | $-O-CH_2-CH_2-O-(CH_2)_3-CH_3$ |
| $CH_3$ | $CH_3$ | F | $-OCHF_2$ | $-O-CH_2-CH=CH-CH_3$ |
| $CH_3$ | $CH_3$ | F | $-OCHF_2$ | $-O-(CH_2-CH_2-O)-CH_3$ |
| $CH_3$ | $CH_3$ | F | $-OCHF_2$ | $-S-CH_2-COOC_2H_5$ |
| $CH_3$ | $CH_3$ | F | $-OCHF_2$ | $-S-CH_2-COO-CH_2-CH_2-OCH_3$ |
| $CH_3$ | $CH_3$ | F | $-OCHF_2$ | $-O-CH_2-CH_2-OC_2H_5$ |

| $R^1$ | $R^2$ | $R^3$ | $R^4$ | $R^5$ |
|---|---|---|---|---|
| $CH_3$ | $CH_3$ | F | $-OCHF_2$ | $-O-CH_2-CH=CH_2$ |
| $CH_3$ | $CH_3$ | F | $-OCHF_2$ | $-O-CH_2-C\equiv CH$ |
| $CH_3$ | $CH_3$ | H | $-OCHF_2$ | $-O-CH_2-CH_2-O-C_2H_5$ |
| $CH_3$ | $CH_3$ | H | $-OCHF_2$ | $-O-CH_2-CH=CH_2$ |
| $CH_3$ | $CH_3$ | H | $-OCHF_2$ | $-O-CH_2-C\equiv CH$ |
| $CH_3$ | $CH_3$ | H | $-OCHF_2$ | $-S-CH_2-COOC_2H_5$ |
| $CH_3$ | $CH_3$ | H | $-OCHF_2$ | $-S-CH_2-COO-CH_2CH_2-OCH_3$ |
| $CH_3$ | $CH_3$ | H | $-OCHF_2$ | $-O-(CH_2-CH_2-O)_2-CH_3$ |
| $CH_3$ | $CH_3$ | H | $-OCHF_2$ | $-O-CH_2-CH=CH-CH_3$ |
| $CH_3$ | $CH_3$ | H | $-OCHF_2$ | $-O-CH_2-CH_2-O-(CH_2)_3-CH_3$ |
| $CH_3$ | $CH_3$ | H | $-OCHF_2$ | $-S-CH(CH_3)-COOC_2H_5$ |
| $CH_3$ | $C_2H_5$ | F | $OCF_3$ | $-O-CH_2-CH_2-O-(CH_2)_3-CH_3$ |
| $CH_3$ | $C_2H_5$ | F | $OCF_3$ | $-O-CH_2-CH=CH-CH_3$ |
| $CH_3$ | $C_2H_5$ | F | $OCF_3$ | $-O-(CH_2-CH_2-O)_2-CH_3$ |
| $CH_3$ | $C_2H_5$ | F | $OCF_3$ | $-S-CH_2-COOC_2H_5$ |
| $CH_3$ | $C_2H_5$ | F | $OCF_3$ | $-S-CH_2-COO-CH_2-CH_2-OCH_3$ |
| $CH_3$ | $C_2H_5$ | F | $OCF_3$ | $-O-CH_2-CH_2-OC_2H_5$ , |
| $CH_3$ | $C_2H_5$ | F | $OCF_3$ | $-O-CH_2-CH=CH_2$ |
| $CH_3$ | $C_2H_5$ | F | $OCF_3$ | $-O-CH_2-C\equiv CH$ |
| $CH_3$ | $CH_3$ | H | $OCF_3$ | $-O-CH_2-CH_2-O-C_2H_5$ |

| $R^1$ | $R^2$ | $R^3$ | $R^4$ | $R^5$ |
|---|---|---|---|---|
| $CH_3$ | $CH_3$ | H | $OCF_3$ | $-O-CH_2-CH=CH_2$ |
| $CH_3$ | $CH_3$ | H | $OCF_3$ | $-O-CH_2-C\equiv CH$ |
| $CH_3$ | $CH_3$ | H | $OCF_3$ | $-S-CH_2-COOC_2H_5$ |
| $CH_3$ | $CH_3$ | H | $OCF_3$ | $-S-CH_2-COO-CH_2CH_2-OCH_3$ |
| $CH_3$ | $CH_3$ | H | $OCF_3$ | $-O-(CH_2-CH_2-O)_2-CH_3$ |
| $CH_3$ | $CH_3$ | H | $OCF_3$ | $-O-CH_2-CH=CH-CH_3$ |
| $CH_3$ | $CH_3$ | H | $OCF_3$ | $-O-CH_2-CH_2-O-(CH_2)_3-CH_3$ |
| $CH_3$ | $CH_3$ | H | $OCF_3$ | $-S-\underset{\underset{CH_3}{\vert}}{CH}-COOC_2H_5$ |

Verwendet man beispielsweise 1-(2,5-Difluor-4-methylphenyl)-3,5-dimethylpyrazol und Salpetersäure als Ausgangsstoffe, so läßt sich der Reaktionsablauf des erfindungsgemäßen Verfahrens (a) durch das folgende Formelschema darstellen:

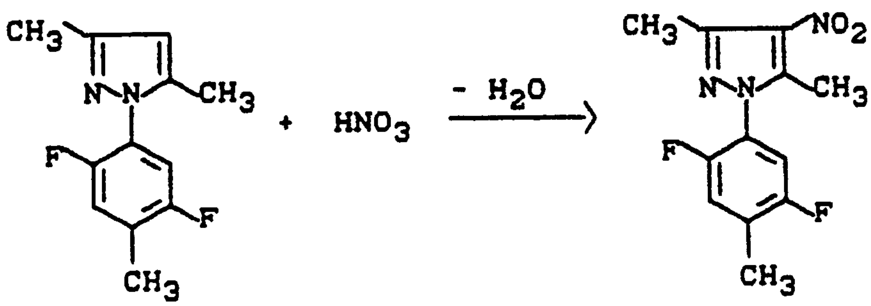


Verwendet man beispielsweise 1-(4-Cyano-2-fluor-5-hydroxyphenyl)-3,5-dimethyl-4-nitropyrazol und Allylbromid als Ausgangsstoffe, so läßt sich der Reaktionsablauf des erfindungsgemäßen Verfahrens (b-α) durch das folgende Formelschema darstellen:

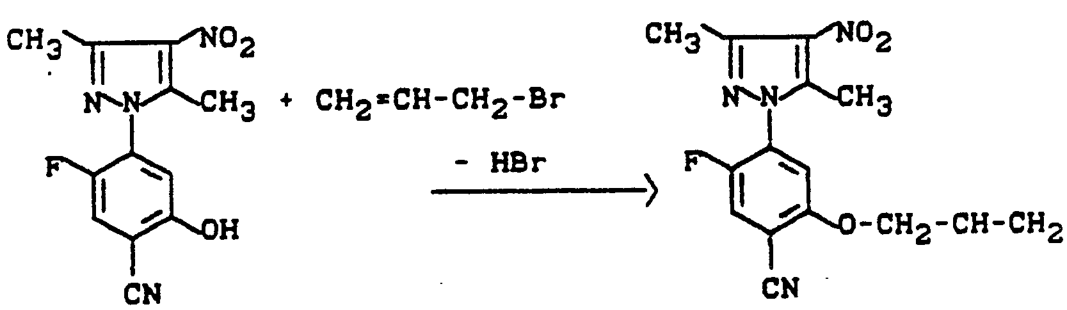


Verwendet man beispielsweise 1-(4-Methyl-5-mercaptophenyl)-3-methyl-4-nitropyrazol und Acetanhydrid als Ausgangsstoffe, so läßt sich der Reaktionsablauf des erfindungsgemäßen Verfahrens (b-ß) durch das folgende Formelschema darstellen:

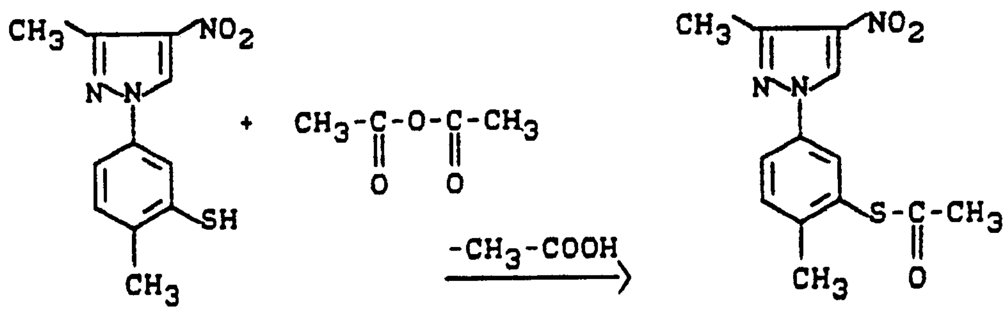


Verwendet man beispielsweise 1-(2-Fluor-5-hydroxy-4-trifluormethylphenyl)-4-nitropyrazol und Methansulfonsäurechlorid als Ausgangsstoffe, so läßt sich der Reaktionsablauf des erfindungsgemäßen Verfahrens (b-γ) durch das folgende Formelschema darstellen:

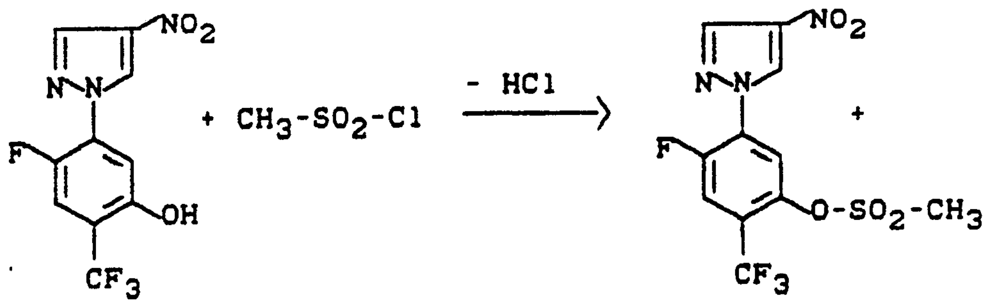


Verwendet man beispielsweise 1-(2,5-Difluor-4-Cyanophenyl)-3,5-dimethyl-4-nitropyrazol und Benzylalkohol als Ausgangsstoffe, so läßt sich der Reaktionsablauf des erfindungsgemäßen Verfahrens (c) durch das folgende Formelschema darstellen:

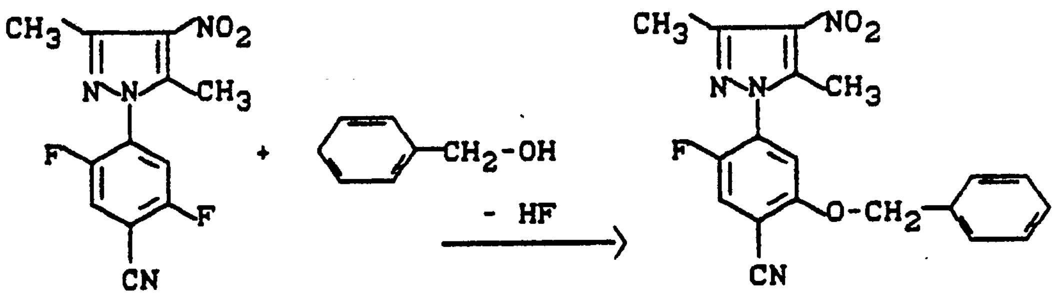


Verwendet man beispielsweise 1-(4-Cyano-5-benzyloxyphenyl)-5-methyl-4-nitropyrazol und molekularen Wasserstoff in Gegenwart eines Hydrierkatalysators als Ausgangsstoffe, so läßt sich der Reaktionsablauf des erfindungsgemäßen Verfahrens (d) durch das folgende Formelschema darstellen:

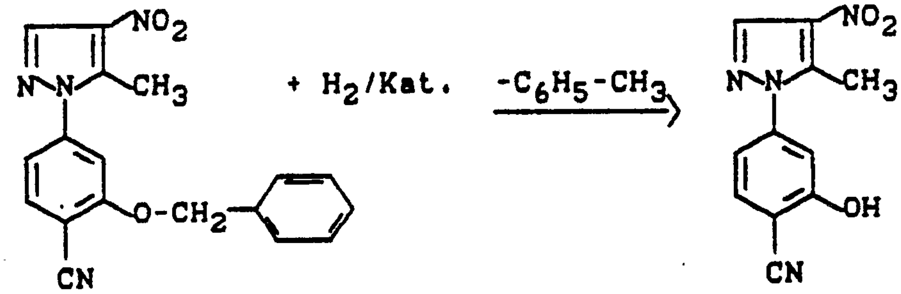


Verwendet man beispielsweise 1-(4-Methyl-5-methansulfonyloxyphenyl)-3,5-bis-(trifluormethyl)-4-nitro-

pyrazol und Natriumhydroxid als Ausgangsstoffe, so läßt sich der Reaktionsablauf des erfindungsgemäßen Verfahrens (e) durch das folgende Formelschema darstellen:

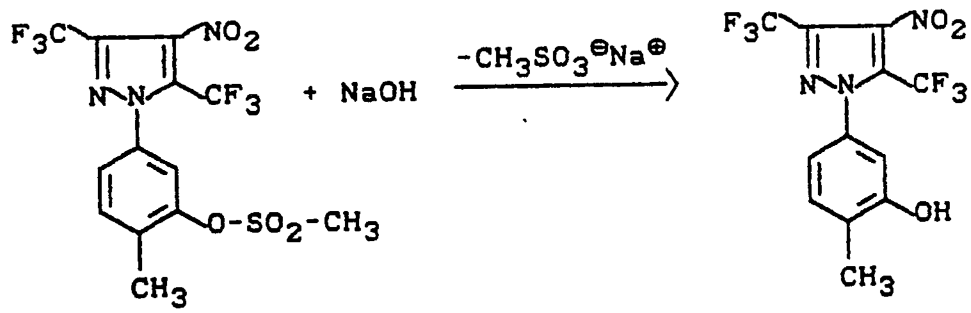


Verwendet man beispielsweise 1-(2,5-Difluor-4-methoxyphenyl)-4-nitropyrazol und Natriumhydrogensulfid als Ausgangsstoffe, so läßt sich der Reaktionsablauf des erfindungsgemäßen Verfahrens (f) durch das folgende Formelschema darstellen:

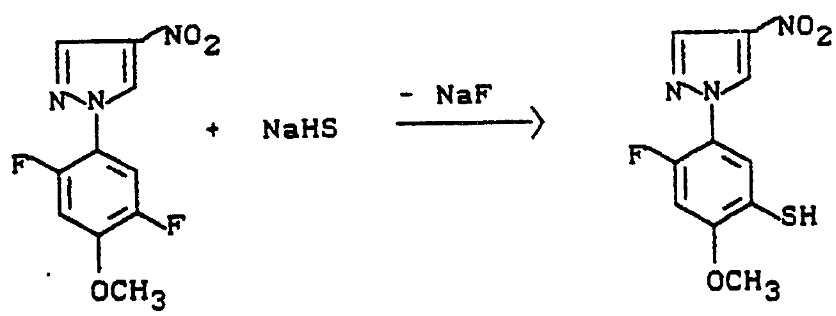


Die zur Durchführung des erfindungsgemäßen Verfahrens (a) als Ausgangsstoffe benötigten 4-unsubstituierten 1-Phenylpyrazole sind durch die Formel (II) allgemein definiert. In dieser Formel (II) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die 4-unsubstituierten 1-Phenylpyrazole der Formel (II) sind teilweise bekannt (vgl. z.B. DE 27 46 067) oder erhältlich in Analogie zu bekannten Verfahren (vgl. z.B. EP-A 200 872), teilweise sind sie Gegenstand der eigenen bisher nicht veröffentlichten deutschen Patentanmeldung P 38 39 480.4 vom 23.11.88.

Man erhält sie beispielsweise, wenn man Arylhydrazine der Formel (V),

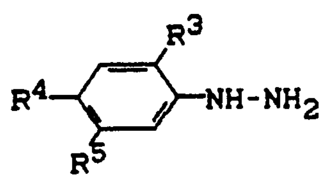 (V)

in welcher
$R^3$, $R^4$ und $R^5$ die oben angegebene Bedeutung haben,
entweder

(α) mit 1,3-Dicarbonylverbindungen der Formel (VI),

$$R^1-\underset{\underset{O}{\|}}{C}-CH_2-\underset{\underset{O}{\|}}{C}-R^2 \quad \text{(VI)}$$

in welcher
$R^1$ und $R^2$ die oben angegebene Bedeutung haben,
oder mit Derivaten dieser 1,3-Dicarbonylverbindungen wie Enolethern, Enaminen, Halogeniden, Acetalen oder Ketalen, gegebenenfalls in Gegenwart eines Verdünnungsmittels wie beispielsweise Ethylenglykolmonoethylether oder Ethanol bei Temperaturen zwischen 50 °C und 150 °C umsetzt (vgl. hierzu insbesondere EP-A 200 872 oder EP-A 286 968);

oder

(ß) mit Acrylesterderivaten der Formel (VII),

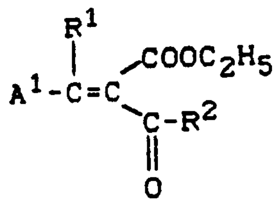

(VII)

in welcher
$A^1$ für Alkoxy oder Dialkylamino, insbesondere für Ethoxy oder Dimethylamino steht und
$R^1$ und $R^2$ die oben angegebene Bedeutung haben,
(oder mit entsprechend substituierten Acrylnitrilderivaten) gegebenenfalls in Gegenwart eines Verdünnungsmittels wie beispielsweise Ethanol bei Temperaturen zwischen 20 °C und 150 °C umsetzt und anschließend die so erhältlichen 4-Pyrazolylcarbonsäureester der Formel (VIII),

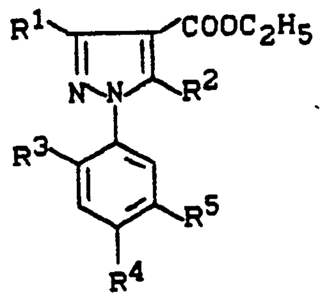

(VIII)

in welcher
$R^1$, $R^2$, $R^3$, $R^4$ und $R^5$ die oben angegebene Bedeutung haben,
(oder die entsprechenden 4-Pyrazolylcarbonitrile) in allgemein üblicher Art und Weise mit wässrigen Säuren oder Basen verseift zu den entsprechenden 4-Pyrazolylcarbonsäuren und diese anschließend ebenfalls in allgemein üblicher Art und Weise thermisch decarboxyliert (vgl. hierzu auch EP-A 200 872).

Derart erhältliche 4-unsubstituierte 1-Phenylpyrazole der Formel (II) können gegebenenfalls anschließend im Arylteil in Analogie zur Durchführung eines oder mehrerer der erfindungsgemäßen Verfahren (b), (c), (d), (e) oder (f) modifiziert werden.

Arylhydrazine der Formel (V) sind bekannt oder erhältlich in Analogie zu bekannten Verfahren (vgl. z.B. EP-A 200 872; BE 71 17 05, DE-OS 34 20 985; JP 60/161 959; DE 27 12 434; J. Indian Chem. Soc. 62, 673-675 [1985]; US 4 224 335; DE 26 27 190; Khim. Geterotsikl. Soedin. 1982, 1215-1219; US 3 778 443 sowie die Herstellungsbeispiele).

1,3-Dicarbonylverbindungen der Formel (VI) bzw. deren Derivate sind allgemein bekannte Verbindungen der organischen Chemie (vgl. z.B. Chem. Ber. 59, 1282 [1926]; Liebigs Ann. Chem. 452, 182 [1927]; J. org. Chemistry 21, 97 [1956]).

Acrylesterderivate der Formel (VII) bzw. die entsprechend substituierten Acrylnitrilderivate sind ebenfalls allgemein bekannte Verbindungen der organischen Chemie.

Die zur Durchführung des erfindungsgemäßen Verfahrens (b) als Ausgangsstoffe benötigten N-Aryl-stickstoffheterocyclen sind durch die Formel (Ib) allgemein definiert. In dieser Formel (Ib) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden. $R^{5-2}$ steht für Hydroxy oder Mercapto.

Die N-Aryl-Sickstoffheterocyclen der Formel (Ib) sind erfindungsgemäße Verbindungen und erhältlich mit Hilfe der erfindungsgemäßen Verfahren (a), (d), (e) und (f).

Die zur Durchführung des erfindungsgemäßen Verfahrens (b-α) weiterhin als Ausgangsstoffe benötigten Alkylierungsmittel sind durch die Formel (IIIa) allgemein definiert. In dieser Formel (IIIa) steht $R^{10}$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für den Substituenten

$R^7$ genannt wurden, mit Ausnahme des Wasserstoffrestes und des Restes $-\underset{\overset{\|}{O}}{C}-R^8$.

$E^1$ steht vorzugsweise für Halogen, insbesondere für Brom oder Iod, für Alkoxysulfonyloxy oder für gegebenenfalls substituiertes Arylsulfonyloxy wie beispielsweise Methoxysulfonyloxy, Ethoxysulfonyloxy oder p-Toluolsulfonyloxy.

Die Alkylierungsmittel der Formel (IIIa) sind allgemein bekannte Verbindungen der organischen Chemie.

Die zur Durchführung des erfindungsgemäßen Verfahrens (b-ß) weiterhin als Ausgangsstoffe benötigten Acylierungsmittel sind durch die Formel (IIIb) allgemein definiert. In dieser Formel (IIIb) steht $R^8$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^2$ steht vorzugsweise für Halogen, insbesondere für Chlor oder Brom, oder für einen Rest $-O-\underset{\overset{\|}{O}}{C}-R^8$,

wobei $R^8$ die oben angegebene Bedeutung hat.

Die Acylierungsmittel der Formel (IIIb) sind allgemein bekannte Verbindungen der organischen Chemie.

Die zur Durchführung des erfindungsgemäßen Verfahrens (b-$\gamma$) weiterhin als Ausgangsstoffe benötigten Sulfonylierungsmittel sind durch die Formel (IIIc) allgemein definiert. In dieser Formel (IIIc) steht $R^9$ vorzugsweise für diejenigen Reste, die bereits im Zusammenhang mit der Beschreibung der erfindungsgemäßen Stoffe der Formel (I) als bevorzugt für diesen Substituenten genannt wurden. $E^2$ steht vorzugsweise für Halogen, insbesondere für Fluor, Chlor oder Brom.

Die Sulfonylierungsmittel der Formel (IIIc) sind ebenfalls allgemein bekannte Verbindungen der organischen Chemie.

Die zur Durchführung der erfindungsgemäßen Verfahren (c) und (f) als Ausgangsstoffe benötigten N-Aryl-Stickstoffheterocyclen sind durch die Formel (Id) allgemein definiert. In dieser Formel (Id) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden. $R^{5-4}$ steht vorzugsweise.für Fluor, Chlor oder Brom,insbesondere für Fluor.

Die N-Aryl-Stickstoffheterocyclen der Formel (Id) sind erfindungsgemäße Verbindungen und erhältlich mit Hilfe des erfindungsgemäßen Verfahrens (a).

Die zur Durchführung des erfindungsgemäßen Verfahrens (c) weiterhin als Ausgangsstoffe benötigten Alkohole oder Thiole sind durch die Formel (IV) allgemein definiert. In dieser Formel (IV) steht $R^{5-3}$ vorzugsweise für einen Rest $-O-R^{10}$ oder für einen Rest $-S-R^{10}$, wobei $R^{10}$ vorzugsweise für diejenigen Reste steht, die bereits im Zusammenhang mit der Beschreibung der Ausgangsstoffe der Formel (IIIa) als bevorzugt für diesen Substituenten genannt wurden.

Die Alkohole oder Thiole der Formel (VI) sind allgemein bekannte Verbindungen der organischen Chemie.

Die zur Durchführung des erfindungsgemäßen Verfahrens (d) als Ausgangsstoffe benötigten N-Aryl-Stickstoffheterocyclen sind durch die Formel (If) allgemein definiert. In dieser Formel (If) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden. $R^{10-1}$ steht vorzugsweise für Allyl oder Benzyl.

Die N-Aryl-Stickstoffheterocyclen der Formel (If) sind erfindungsgemäße Verbindungen und erhältlich mit Hilfe der erfindungsgemäßen Verfahren (a), (b-$\alpha$) oder (c).

Die zur Durchführung des erfindungsgemäßen Verfahrens (e) als Ausgangsstoffe benötigten N-Aryl-Stickstoffheterocyclen sind durch die Formel (Ig) allgemein definiert. In dieser Formel (Ig) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{5-5}$ steht vorzugsweise für einen Rest $-O-\underset{\overset{\|}{O}}{C}-R^8$

oder $-O-SO_2-R^9$, wobei $R^8$ und $R^9$ vorzugsweise für diejenigen Reste stehen, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (Ig) sind erfindungsgemäße Verbindungen und erhältlich mit Hilfe der erfindungsgemäßen Verfahren (a), (b-ß) oder (b-$\gamma$).

Als Nitrierungsmittel zu Durchführung des erfindungsgemäßen Verfahrens (a) kommen alle üblichen Nitrierungsmittel in Frage. Vorzugsweise verwendet man konzentrierte Salpetersäure oder Nitriersäure.

Als Verdünnungsmittel zur Durchführung des erfindungsgemäßen Verfahrens (a) kommen alle üblicherweise für derartige Nitrierungsreaktionen verwendbaren Lösungsmittel in Frage. Vorzugsweise verwendet man die als Reagenzien in Frage kommenden Säuren oder Gemische, wie beispielsweise Schwefelsäure,

Salpetersäure oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Katalysatoren oder Reaktionshilfsmittel zur Durchführung des erfindungsgemäßen Verfahrens (a) kommen ebenfalls die für derartige Reakionen üblichen Katalysatoren in Frage; vorzugsweise verwendet man saure Katalysatoren wie beispielsweise Schwefelsäure oder Acetanhydrid.

Die Reaktionstemperaturen können bei der Durchführung des erfindungsgemäßen Verfahrens (a) in einem größeren Bereich variiert werden. Im allgemeinen arbeitet man zwischen —50 °C und +200 °C, vorzugsweise zwischen -20 °C und +150 °C.

Zur Durchführung des erfindungsgemäßen Verfahrens (a) setzt man pro Mol an 4-unsubstituiertem 1-Phenylpyrazol der Formel (II) im allgemeinen 1.0 bis 10.0 Mol, vorzugsweise 1.0 bis 5.0 Mol an Nitrierungsmittel und gegebenenfalls 0.1 bis 10 Mol, vorzugsweise 0.5 bis 5.0 Mol an Reaktionshilfsmittel ein.

Die Reaktionsdurchführung, Aufarbeitung und Isolierung der Reaktionsprodukte erfolgt nach allgemein üblichen Methoden.

Zur Durchführung des erfindungsgemäßen Verfahrens (b) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner in den Varianten (b-α), (b-ß) oder (b-γ) Verbindungen der Formeln (IIIa), (IIIb) oder (IIIc) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (b) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (b) setzt man pro Mol an N-Aryl-Stickstoffheterocyclus der Formel (Ib) in den Varianten (b-α), (b-ß) oder (b-γ) im allgemeinen jeweils 1,0 bis 20:0 Mol, vorzugsweise jeweils 1,0 bis 15,0 Mol, an Alkylierungsmittel der Formel (IIIa) oder Acylierungsmittel der Formel (IIIb) oder Sulfonylierungsmittel der Formel (IIIc) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (Ia) erfolgt nach üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid. Es ist auch möglich die als Reaktionspartner infrage kommenden Alkohole oder Thiole der Formel (IV) in einem entsprechenden Überschuß gleichzeitig als Verdünnungsmittel einzusetzen.

Das erfindungsgemäße Verfahre (c) wird vorzugsweise in Gegenwart eines geeigneten Reaktionshilfsmittels durch geführt. Als solche kommen alle üblicherweise verwendbaren anorganischen und organischen Basen in Frage. Vorzugsweise verwendet man Alkalimetallhydride, -hydroxide, -amide, -alkoholate oder -carbonate wie beispielsweise Natriumhydrid, Natriumamid, Natriumhydroxid, Kaliumhydroxid, Natriummethylat, Natriumethylat, Kalium-t-butylat oder Kaliumcarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (c) setzt man pro Mol an N-Aryl-Stickstoffheterocyclus der Formel (Id) im allgemeinen 1.0 bis 3.0 Mol, vorzugsweise 1.0 bis 1.5 Mol an Alkohol oder Thiol der Formel (IV) und gegebenenfalls 1.0 bis 3.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (d) kommen alle unter Hydrierungsbe dingungen inerten üblichen Solventien in Betracht. Vorzugsweise verwendbar sind Kohlenwasserstoffe, wie Petrolether, Pentan, Hexan, Heptan, Cyclohexan, Alkohole, wie Methanol, Ethanol, n-Propanal und i-Propanol, Ether, wie Diethylether, Dioxan, Tetrahydrofuran oder Ethylenglykoldimethyl- oder -diethylether, Carbonsäuren, wie Essigsäure oder Propionsäure oder Gemische dieser Verdünnungsmittel miteinander oder mit Wasser. Es kann jedoch auch ohne Verdünnungsmittel gearbeitet werden.

Das erfindungsgemäße Verfahren (d) kann gegebenenfalls in Gegenwart eines geeigneten Reaktionshilfsmittels durchgeführt. Als solche kommen alle üblichen anorganischen oder organischen Basen in Frage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Als Hydrierkatalysatoren können bei dem erfindungsgemäßen Verfahren (d) alle üblicherweise für derartige Reaktionen verwendbaren Katalysatoren eingesetzt werden. Vorzugsweise verwendet man Raney-Nickel oder Edelmetallkatalysatoren, wie Palladium, Ruthenium, Palladiumoxid, Platin oder Platinoxid, gegebenenfalls auf einem geeigneten Trägerstoff, wie z.B. Kohle, Aluminiumoxid oder Siliciumdioxid.

Als Säuren setzt man bei der Durchführung des erfindungsgemäßen Verfahrens (d) alle üblichen für derartige Isomerisierungsreaktionen verwendbaren Säuren ein. Vorzugsweise verwendet man anorganische Protonensäuren wie Salzsäure, Schwefelsäure oder Salpetersäure, organische aliphatische oder aromatische Carbonsäuren, wie beispielsweise Essigsäure, Propionsäure oder Benzoesäure oder übliche saure Ionenaustauscher.

Als Isomerisierungskatalysatoren kommen bei der Durchfürung des erfindungsgemäßen Verfahrens (d) übliche Doppelbindungsisomerisierungskatalysatoren infrage. Vorzugsweise verwendet man Edelmetallkomplexverbindungen, wie beispielsweise Tris-(Triphenylphospin)-Rhodiumchlorid oder Tris-(Triphenylphosphin)-Palladiumchlorid.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 150 °C, vorzugsweise bei Temperaturen zwischen 20 °C und 120 °C.
Das erfindungsgemäße Verfahren (d) kann bei Normaldruck oder aber unter erhöhtem Druck bis 200 atm durchgeführt werden.

Zur Durchführung des erfindungsgemäßen Verfahrens (d) setzt man pro Mol an N-Aryl-Stickstoffheterocyclus der Formel (If) für den Fall, daß $R^{10-1}$ für Benzyl steht, im allgemeinen 1.0 bis 5.0 Mol, vorzugsweise 1.0 bis 3.0 Mol an Wasserstoff und gegebenenfalls 0.001 bis 1.0 Mol, vorzugsweise 0.01 bis 0.5 Mol an Hydrierkatalysator ein.
Die Reaktionsdurchführung, Aufarbeitung und Isolierung der Reaktionsprodukte erfolgt nach allgemein üblichen Methoden.

Zur Durchführung des erfindungsgemäßen Verfahrens (d) setzt man pro Mol an N-Aryl-Stickstoffheterocyclus der Formel (If) für den Fall, daß $R^{10-1}$ für Allyl steht, im allgemeinen 1.0 bis 20.0 Mol, vorzugsweise 1.0 bis 10.0 Mol an Säure und 0.001 bis 1.0 Mol, vorzugsweise 0.05 bis 0.5 Mol an Isomerisierungskatalysator sowie gegebenenfalls 0.001 bis 5.0 Mol, vorzugsweise 0.01 bis 3.0 Mol an Reaktionshilfsmittel ein.
Die Reaktionsdurchführung, Aufarbeitung und Isolierung der Reaktionsprodukte erfolgt nach allgemein üblichen Methoden.

Zur Durchführung des erfindungsgemäßen Verfahrens (e) kommen insbesondere verdünnte anorganische Protonensäuren, wie insbesondere Chlorwasserstoffsäure oder Schwefelsäure oder wässrige Lösungen von anorganischen Basen, wie beispielsweise wässrige Lösungen von Natriumhydroxid, Kaliumhydroxid, Natriumcarbonat oder Kaliumcarbonat infrage.

Als Verdünnungsmittel zur Durchführung des erfindungsgemäßen Verfahrens (e) kommen anorganische oder organische polare Lösungsmittel in Frage. Vorzugsweise verwendet man Alkohole, wie beispielsweise Methanol, Ethanol oder Propanol, oder deren Gemische mit Wasser.

Die Reaktionstemperaturen können bei der Durchführung des erfindungsgemäßen Verfahrens (e) in einem größeren Bereich variiert werden. Im allgemeinen arbeitet man bei Temperaturen zwischen 20 °C

und 150 °C, vorzugsweise bei Temperaturen zwischen 50 °C und 120 °C.

Zur Durchführung des erfindungsgemäßen Verfahrens (e) setzt man pro Mol an N-Aryl-Stickstoffheterocyclus der Formel (Ig) im allgemeinen 1.0 bis 20.0 Mol, vorzugsweise 1.0 bis 10.0 Mol an Säure oder Base ein und erwärmt für mehrere Stunden auf die erforderliche Reaktionstemperatur. Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (f) kommen anorganische oder organische polare Lösungsmittel infrage. Vorzugsweise verwendet man die bei Verfahren (e) genannten Verdünnungsmittel.

Das erfindungsgemäße Verfahren (f) wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (f) in einem größeren Bereich variiert werden. Im allgemeinen arbeitet man bei Temperaturen zwischen - 20 °C und 120 °C, vorzugsweise bei Temperaturen zwischen 0 °C und 50 °C.

Zur Durchführung des erfindungsgemäßen Verfahrens (f) setzt man pro Mol an N-Aryl-Stickstoffheterocyclus der Formel (Id) im allgemeinen 1.0 bis 5.0 Mol, vorzugsweise 1.0 bis 1.5 Mol an Natriumhydrogensulfid und gegebenenfalls 1.0 bis 5.0 Mol, vorzugsweise 1.0 bis 1.5 Mol an Reaktionshilfsmittel ein. Die Reaktionsdurchführung, Aufarbeitung und Isolierung der Reaktionsprodukte erfolgt nach allgemein üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung von mono- und dikotylen Unkräutern in mono- und dikotylen Kulturen, wie beispielsweise Soja oder Weizen einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln

Im Falle der Benutzung von Wasser als Streckmittel können z.B. auch organische Lösungsmittel als

Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin- 2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D);
2,4-Dichlorphenoxypropionsäure (2,4-DP);
4-(2,4-Dichlorphenoxy)-buttersäure (2,4-DB);
(2-Methyl-4-chlorphenoxy)-essigsäure (MCPA);
(4-Chlor-2-methylphenoxy)-propionsäure (MCPP);
[(4-Amino-3,5-dichlor-6-fluor-2-pyridinyl)-oxy]-essigsäure bzw. deren 1-Methylheptylester (FLUROXYPYR);
2-[4-(2,4-Dichlorphenoxy)-phenoxy]-propionsäure deren Methyl-oder deren Ethylester (DICLOFOP-[METHYL]);
2-{4-[(6-Chlor-2-benz-ox-azo-lyl)-oxy]-phen-oxy}propansäure, deren Methyl- oder deren Ethylester-(FENOXAPROP);
2-Chlor-N-(2,6-dimethylphenyl)-N-[(1H)-pyrazol-1-yl-methyl]-acetamid (METAZACHLOR);
Chloressigsäure-N-(methoxymethyl)-2,6-diethylanilid (ALACHLOR);
2-Ethyl-6-methyl-N-(1-methyl-2-methoxyethyl)chloracetanilid (METOLACHLOR);
N-(1-Ethylpropyl)-3,4-dimethyl-2,6-dinitroanilin (PENDIMETHALIN);
2,6-Dinitro-4-trifluormethyl-N,N-dipropylanilin (TRIFLURALIN);
5-(2-Chlor-4-trifluormethyl-phenoxy)-2-nitra-benzoesäure (ACIFLUORFEN);
Methyl-5-(2,4-dichlorphenoxy)-2-nitrobenzoat (BIFENOX);
5-(2-Chlor-4-trifluormethyl-phenoxy)-N-methylsulfonyl-2-nitrobenzamid (FOMESAFEN);
N,N-Dimethyl-N'-(3-chlor-4-methylphenyl)-harnstoff (CHLORTOLURON);
N,N-Dimethyl-N'-(4-isopropylphenyl)-harnstoff (ISOPROTURON);
2-[4,5-Dihydro-4-methyl-4-isopropyl-5-oxo-(1H)-imidazol-2-yl]-5-ethyl-pyridin-3-carbonsäure (IMAZETHAPYR);
Methyl-2-[4,5-dihydro-4-methyl-4-(1-methylethyl)-5-axo-1H-imidazol-2-yl]-4(5)-methylbenzoat (IMAZAMETHABENZ);

2-[5-Methyl-5-(1-methylethyl)-4-oxo-2-imidazolin-2-yl]-3-chinolincarbonsäure (IMAZAQUIN);
3,5-Dibrom-4-hydroxy-benzonitril (BROMOXYNIL);
3,5-Diiod-4-hydroxybenzonitril (IOXYNIL);
N-Methyl-2-(1,3-benzthiazol-2-yloxy)-acetanilid (MEFENACET);
Ethyl-2-{[(4-chlor-6-methoxy-2-pyrimidinyl)-aminocarbonyl]-aminosulfonyl}-benzoat (CHLORIMURON);
2-Chlor-N-{[(4-methoxy-6-methyl-1,3,5-triazin-2-yl)-amino]-carbonyl]-benzolsulfonamid (CHLORSULFURON);
2-{[[((4-Methoxy-6-methyl-1,3,5-triazin-2-yl)-amino)carbonyl]-amino]-sulfonyl}-benzoesäure oder deren Methylester (METSULFURON);
3-[[[[(4-Methoxy-6-methyl-1,3,5-triazin-2-yl)-amino]carbonyl]-amino]-sulfonyl]-thiophen-2-carbonsäuremethylester (THIAMETHURON);
N,N-Diisopropyl-S-(2,3,3-trichlorallyl)-thiolcarbamat (TRIALLATE);
4-Ethylamino-2-t-butylamino-6-methylthio-s-triazin (TERBUTRYNE);
3-Isopropyl-2,1,3-benzothiadiazin-4-on-2,2-dioxid (BENTAZON);
4-Amino-6-t-butyl-3-ethylthio-1,2,4-triazin-5(4H)-on (ETHIOZIN);
2-[(2-Chlorphenyl)-methyl]-4,4-dimethylisoxazolidin-3-on (DIMETHAZONE) und/oder O-(6-Chlor-3-phenyl-pyridazin-4-yl)-S-octyl-thiocarbonat (PYRIDATE) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Baden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

## Herstellungsbeispiele

### Beispiel 1

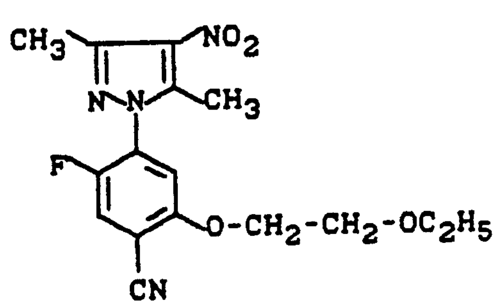

(Verfahren a)

Zu 3,0 g (0,01 Mol) 1-[4-Cyano-2-fluor-5-(2-ethoxy-ethoxy-phenyl]-3,5-dimethylpyrazol in 20 ml Eisessig gibt man zunächst 1 ml Acetanhydrid und anschließend bei 10 °C 0,5 ml (0,011 Mol) 98prozentige Salpetersäure, rührt dann 15 Stunden bei Raumtemperatur, engt zur Trockne ein, nimmt den Rückstand in Wasser auf, extrahiert mehrfach mit Dichlormethan, wäscht die vereinigten organischen Phasen mit gesättigter wässriger Natriumchloridlösung, trocknet über Natriumsulfat, engt im Vakuum ein und reinigt den Rückstand durch Chromatographie an Kieselgel (Laufmittel: Dichlormethan/Methanol 40:1).

Man erhält 0,6 g (17 % der Theorie) an 1-[4-Cyano-2-fluor-5-(2-ethoxyethoxy-phenyl]-3,5-dimethyl-4-nitropyrazol vom Schmelzpunkt 93 °C.

Beispiel 2

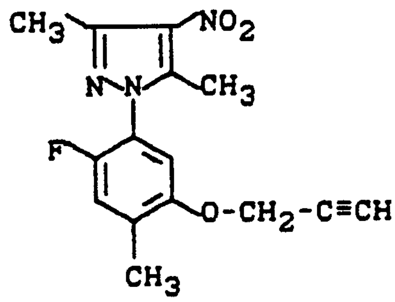

(Verfahren b-α)

Zu 1,3 g (0,005 Mol) 1-(2-Fluor-5-hydroxy-4-methylphenyl)-3,5-dimethyl-4-nitropyrazol und 0,8 g (0,0055 Mol) Kaliumcarbonat in 50 ml Acetonitril gibt man bei Raumtemperatur 0,6 g (0,00525 Mol) Propargylbromid, rührt anschließend 20 Stunden bei 60 °C, engt dann im Vakuum ein, nimmt den Rückstand in Dichlormethan auf, wäscht mit Wasser, trocknet über Natriumsulfat, engt im Vakuum ein und reinigt den Rückstand durch Verrühren mit Petrolether.

Man erhält 1,1 g (73 % der Theorie) an 1-(2-Fluor-5-propargyloxy-4-methylphenyl)-3,5-dimethyl-4-nitropyrazol vom Schmelzpunkt 132 °C.

Beispiel 3

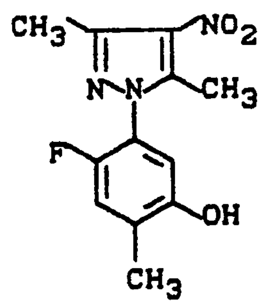

(Verfahren e)

Zu 3,4 g (0,01 Mol) 1-(2-Fluor-5-methansulfonyloxy-4-methylphenyl)-3,5-dimethyl-4-nitropyrazol in 20 ml Ethanol gibt man 20 ml (0,04 Mol) 2 Normale Natronlauge, rührt 2 Stunden bei 50 °C, engt dann im Vakuum ein, nimmt den Rückstand in Wasser auf, stellt mit 2 Normaler Salzsäure einen pH-Wert von 1 bis 2 ein, rührt über Nacht bei Raumtemperatur, saugt den ausgefallenen Niederschlag ab, wäscht mit Wasser neutral und trocknet.

Man erhält 2,3 g (86,8 % der Theorie) an 1-(2-Fluor-5-hydroxy-4-methylphenyl)-3,5-dimethyl-4-nitropyrazol vom Schmelzpunkt 147 °C.

Beispiel 4

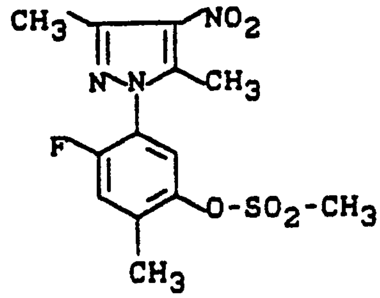

(Verfahren a)

Zu 3,0 g (0,01 Mol) 1-(2-Fluor-5-methansulfonyloxy-4-methylphenyl)-3,5-dimethylpyrazol in 20 ml konzentrierter Schwefelsäure gibt man bei 10 °C tropfenweise unter Rühren 1 ml (0,022 Mol) 98prozentige Salpetersäure, rührt anschließend 4 Stunden bei Raumtemperatur, gibt dann die Reaktionsmischung in Wasser, saugt ausgefallenen Niederschlag ab und trocknet ihn.

Man erhält 2,4 g (70 % der Theorie) an 1-(2-Fluor-5-methansulfonyloxy-4-methylphenyl)-3,5-dimethyl-4-nitropyrazol vom Schmelzpunkt 138 °C (Zersetzung).

Beispiel 5

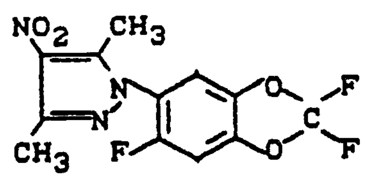

(Verfahren a)

Zu 2,2 g (0,01 Mol) 1-(2-Fluor-4,5-difluordioxymethylenphenyl-3,5-dimethylpyrazol in 30 ml Eisessig gibt man 1,7 ml Acetanhydrid und anschließend bei 10 °C 0,7 ml (0,015 Mol) 98prozentige Salpetersäure, rührt dann 15 Stunden bei Raumtemperatur, engt zur Trockne ein, nimmt den Rückstand in Wasser auf, extrahiert mehrfach mit Dichlormethan, wäscht die vereinigten organischen Phasen mit gesättigter wässriger Natriumchloridlösung und anschließend mit wässriger Natriumhydrogencarbonatlösung, trocknet über Natriumsulfat, engt im Vakuum ein und reinigt den Rückstand durch Chromatographie an Kieselgel (Laufmittel: Dichlormethan/Methanol 40:1).

Man erhält 0,8 g (25 % der Theorie) an 1-(2-Fluor-4,5-difluordioxymethylenphenyl)-3,5-dimethyl-4-nitropyrazol vom Schmelzpunkt 105-114 °C.

In entsprechender Weise und gemäß den allgemeinen Angaben zur Herstellung erhält man die folgenden N-Aryl-Stickstoffheterocyclen der allgemeinen Formel (I):

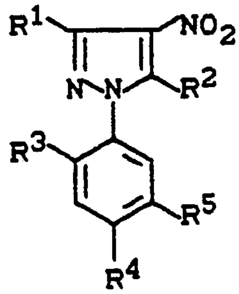

(I)

| Bsp. Nr. | $R^1$ | $R^2$ | $R^3$ | $R^4$ | $R^5$ | Schmelzpunkt °C |
|---|---|---|---|---|---|---|
| 6 | $CH_3$ | $CH_3$ | F | CN | $-O-CH_2-CH_2-OCH_3$ | $^1$H-NMR*): 2,6; 3,5; 3,8; 4,3; 7,1-7,55 |
| 7 | $CH_3$ | $CH_3$ | F | CN | F | 132 (Zers.) |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel II-1

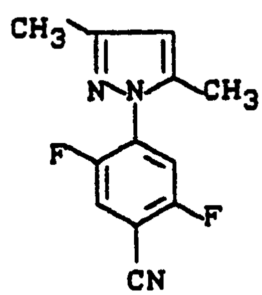

21,1 g (0,125 Mol) 4-Cyano-2,5-difluorphenylhydrazin und 12,5 g (0,125 Mol) 2,4-Pentandion werden in 250 ml Ethanol 2 Stunden bei Raumtemperatur gerührt, anschließend für 15 Stunden auf 70 °C erhitzt und dann im Vakuum eingeengt. Der Rückstand wird mit Petrolether verrührt und abgesaugt.

Man erhält 28 g (96 % der Theorie) an 4-(3,5-Dimethyl-1-pyrazolyl)-2,5-difluorbenzonitril vom Schmelz-

punkt 122 °C.

Beispiel II-2

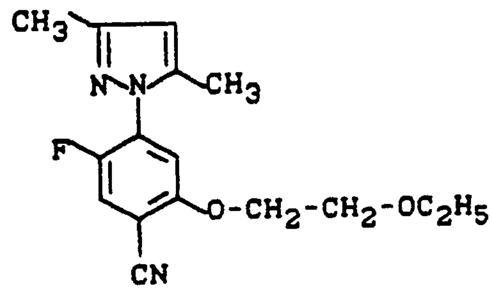

Zu 2,3 g (0,01 Mol) 1-(4-Cyano-2,5-difluorphenyl)-3,5-dimethylpyrazol in 90 ml Ethylenglykolmonoethylether gibt man bei 0 °C 0,8 g (0,025 Mol) Natriumhydrid, rührt dann 15 Stunden bei Raumtemperatur, gießt die Reaktionsmischung in Wasser, rührt 30 Minuten bei Raumtemperatur, saugt ausgefallenen Niederschlag ab und trocknet ihn.

Man erhält 2,5 g (83 % der Theorie) an 1-[4-Cyano-2-fluor-5-(2-ethoxyethoxy-phenyl]-3,5-dimethylpyrazol vom Schmelzpunkt 103 °C.

Beispiel II-3

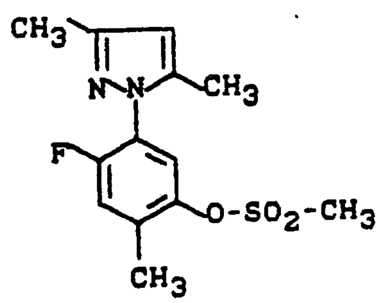

Zu 9,8 g (0,042 Mol) 2-Fluor-4-methyl-5-methansulfonyloxyphenylhydrazin in 150 ml Ethanol gibt man 4,2 g (0,042 Mol) 2,4-Pentandion, erhitzt für 20 Stunden auf Rückflußtemperatur, kühlt ab, engt im Vakuum ein, nimmt den Rückstand in Dichlormethan auf, wäscht mit Wasser, trocknet über Natriumsulfat und entfernt das Lösungsmittel im Vakuum.

Man erhält 10,5 g (84 % der Theorie) an 1-(2-Fluor-5-methansulfonyloxy-4-methylphenyl)-3,5-dimethylpyrazol als Öl.

$^1$H-NMR ($CDCl_3$/Tetramethylsilan):

$\delta$ = 7,4 (1H,d); 7,13 (1H, d); 6,0 (1H,s); 3,22 (3H,s); 2,41 (3H,s); 2,3 (3H,s); 2,2 (3H,s) ppm.

Beispiel II-4

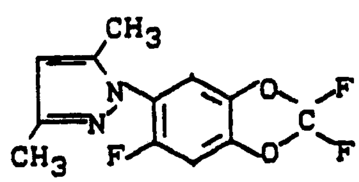

Zu 4,0 g (0,02 Mol) 2-Fluor-4,5-dioxydifluormethylenphenylhydrazin in 100 ml Ethanol gibt man 2,0 g (0,02 Mol) 2,4-Pentandion, erhitzt für 20 Stunden auf Rückflußtemperatur, kühlt auf 60 °C ab, gibt 1 ml konzentrierte Schwefelsäure zu und rührt weitere 4 Stunden bei 60 °C. Zur Aufarbeitung wird abgekühlt, mit Wasser verdünnt, mehrfach mit Dichlormethan extrahiert, die vereinigten organischen Phasen mit wässriger Natriumhydrogencarbonatlösung gewaschen, über Natriumsulfat getrocknet, eingeengt und durch Chromatographie an Kieselgel (Laufmittel: Dichlormethan) gereinigt.

Man erhält 2,4 g (44 % der Theorie) an 1-(2-Fluor-4,5-dioxydifluormethylenphenyl)-3,5-dimethylpyrazol vom Schmelzpunkt 91 °C - 92 °C.

In entsprechender Weise und gemäß den allgemeinen Angaben zur Herstellung erhält man die folgenden Vorprodukte der allgemeinen Formel (II):

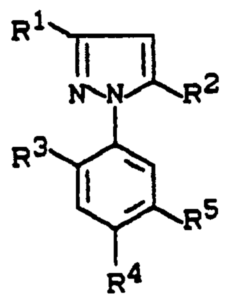

(II)

| Bsp. Nr. | $R^1$ | $R^2$ | $R^3$ | $R^4$ | $R^5$ | Schmelzpunkt °C |
|---|---|---|---|---|---|---|
| II-5 | $CH_3$ | $CH_3$ | F | $CF_3$ | F | 58 |
| II-6 | $CH_3$ | $CH_3$ | F | $CF_3$ | $-O-CH_2-CH_2-OC_2H_5$ | 98 |
| II-7 | $CH_3$ | $CH_3$ | F | $CF_3$ | $-O-CH_2-CH=CH_2$ | 78 |

Beispiel V-1

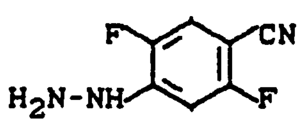

Zu 30 g (0,19 Mol) 2,4,5-Trifluorbenzonitril (vgl. z.B. EP-A 191 181) in 120 ml Ethanol gibt man 11 g (0,22 Mol) Hydrazinhydrat, erhitzt für 2 Stunden auf Rückflußtemperatur, kühlt ab auf Raumtemperatur, engt im Vakuum ein, verrührt den Rückstand mit 50 ml Wasser, saugt ausgefallenes Produkt ab und trocknet.

Man erhält 24 g (75 % der Theorie) an 4-Cyano-2,5-difluorphenylhydrazin vom Schmelzpunkt 158 °C.

Beispiel V-2

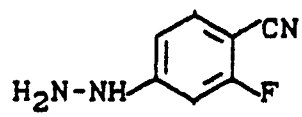

Zu 90 g (0,65 Mol) 2,4-Difluorbenzonitril (vgl. z.B. EP-A 122 693) in 300 ml Methanol gibt man tropfenweise unter Rühren 45 g (0,9 Mol) Hydrazinhydrat, erhitzt für 3 Stunden auf Rückflußtemperatur, engt dann im Vakuum ein, verrührt den Rückstand mit 300 ml Wasser, saugt ab und trocknet.

Man erhält 73 g (74 % der Theorie) an 4-Cyano-3-fluorphenylhydrazin vom Schmelzpunkt 136 °C.

Beispiel V-3

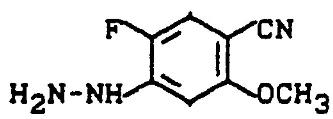

Zu 13 g (0,076 Mol) 4-Cyano-2,5-difluorphenylhydrazin in 100 ml Methanol gibt man 5 g (0,125 Mol) gepulvertes Natriumhydroxid, erhitzt dann für 6 Stunden auf Rückflußtemperatur, engt anschließend im Vakuum ein, gibt den Rückstand in 50 ml Wasser, neutralisiert durch tropfenweise Zugabe von Essigsäure, saugt ausgefallenen Feststoff ab und kristallisiert aus Toluol um.

Man erhält 9 g (65 % der Theorie) an 4-Cyano-2-fluor-5-methoxyphenylhydrazin vom Schmelzpunkt 155-156 °C.

Beispiel V-4

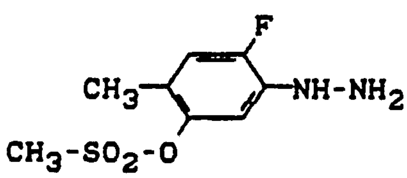

Zu 100 g (0,46 Mol) 2-Fluor-4-methyl-5-methansulfonyloxyanilin in einer Mischung 210 ml Wasser und 93 ml konzentrierter Salzsäure, gibt man bei 5 °C unter Rühren und Kühlung innerhalb von 15 Minuten 95 ml (0,5 Mol) einer 30prozentigen wässrigen Natriumnitritlösung, rührt nach beendeter Zugabe eine weitere Stunde bei 5 °C, gibt dann tropfenweise unter Rühren und Kühlung bei 10 °C im Verlauf von 3 Stunden eine Lösung aus 263 ml konzentrierter Salzsäure und 300 g (1,3 Mol) Zinndichlorid-Dihydrat zu, rührt eine weitere Stunde bei 10 °C und 2 Stunden bei Raumtemperatur, gibt unter Kühlung langsam konzentrierte Natronlauge zu, bis sich ein pH-Wert von 8 einstellt (ca. 420 ml), rührt 3 Stunden bei Raumtemperatur, saugt ausgefallenen Niederschlag ab, wäscht den Rückstand 3 mal mit jeweils 1000 ml Dichlormethan, trocknet die vereinigten organischen Phasen über Magnesiumsulfat und entfernt das Lösungsmittel im Vakuum.

Man erhält 72,4 g (68 % der Theorie) an 2-Fluor-4-methyl-5-methansulfonyloxyphenylhydrazin vom Schmelzpunkt 84 °C bis 91 °C.

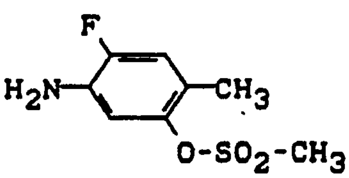

25 g (0,1 Mol) Methansulfonsäure-(4-fluor-2-methyl-5-nitrophenyl)-ester in 150 ml Ethoxyethanol werden mit 4 g Platin auf Aktivkohle (1 %ig) versetzt und bei Raumtemperatur und Normaldruck bis zur Konstanz der Wasserstoffaufnahme hydriert. Zur Aufarbeitung wird der Katalysator abfiltriert und das Lösungsmittel im Vakuum entfernt.

Man erhält 19,2 g (87,7 % der Theorie) an Methansulfonsäure-(4-fluor-2-methyl-5-aminophenyl)-ester vom Schmelzpunkt 66 °C - 69 °C.

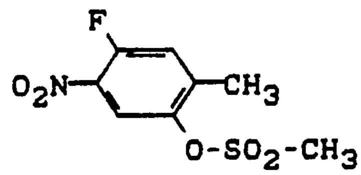

20 g (0,117 Mol) 4-Fluor-2-methyl-5-nitrophenol in 40 ml Pyridin werden bei 10 °C unter Rühren tropfenweise mit 13,4 ml (0,117 Mol) Methansulfonsäurechlorid versetzt, anschließend im Vakuum eingeengt, der Rückstand in Dichlormethan aufgenommen und nacheinander mit verdünnter Salzsäure und Wasser gewaschen, über Magnesiumsulfat getrocknet und im Vakuum vom Lösungsmittel befreit.

Man erhält 26,5 g (91 % der Theorie) an Methansulfonsäure-(4-fluor-2-methyl-5-nitrophenyl)-ester vom Schmelzpunkt 96 °C - 97 °C.

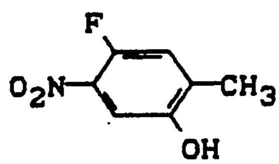

156 g (0,68 Mol) Methyl-(4-fluor-2-methyl-5-nitrophenyl)-carbonat werden in einer Mischung aus 400 ml Dioxan und 300 ml kanzentrierter Salzsäure 12 Stunden auf Rückflußtemperatur erhitzt, anschließend abgekühlt, im Vakuum eingeengt und der Rückstand in Methyl-t-butylether aufgenommen. Man extrahiert mit 400 ml 10prozentiger Natronlauge, säuert die wässrige Phase an, filtriert den ausgefallenen Feststoff ab und kristallisiert aus Toluol um.

Man erhält 86 g (73,5 % der Theorie) an 4-Fluor-2-methyl-5-nitrophenol vom Schmelzpunkt 138 °C - 142 °C.

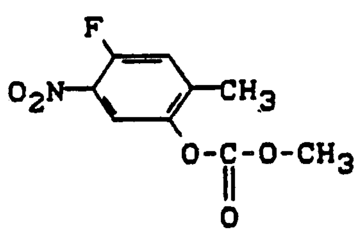

65 g (0,35 Mol) (2-Methyl-4-fluorphenyl)-methylcarbonat in 130 ml Dichlormethan werden bei 20 °C tropfenweise unter Rühren mit 65 g eines Gemisches aus konzentrierter Salpetersäure und konzentrierter Schwefelsäure (1:2) versetzt, anschließend 2 Stunden bei 20 °C und eine weitere Stunde bei 30 °C gerührt, abgekühlt, auf Eis gegossen, mit Dichlormethan extrahiert und destilliert.

Man erhält 62 g (76,7 % der Theorie) an 5-Fluor-2-methoxycarbonyloxy-4-nitrotoluol vom Siedepunkt 120 °C - 125 °C bei 0,27 mbar und vom Schmelzpunkt 68 °C.

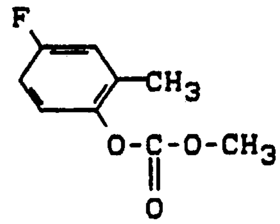

Zu 42 g (1,05 Mol) Natriumhydroxid in 660 ml Wasser gibt man 106 g (0,84 Mol) 2-Methyl-4-fluorphenol (vgl. z.B. EP 18 606), tropft anschließend unter Rühren bei 5 °C 108 g (1,14 Mol) Chlorameisensäuremethylester zu, rührt nach beendeter Zugabe weitere 2 Stunden bei 5 °C, extrahiert mit Dichlormethan, trocknet über Natriumsulfat und destilliert im Vakuum.

Man erhält 139 g (88 % der Theorie) an Methyl-(4-fluor-2-methylphenyl)-carbonat vom Siedepunkt 98 °C bis 100 °C bei 16 mbar und vom Brechungsindex $n_D^{20}$ 1,4770.

Beispiel V-5

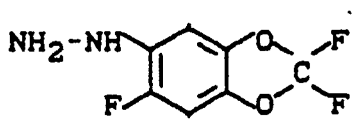

Zu 60 g (0,314 Mol) 2-Fluor-4,5-dioxydifluormethylenanilin in einer Mischung aus 145,5 ml Wasser und 63,7 ml konzentrierter Salzsäure, gibt man bei 5 °C unter Rühren und Kühlung innerhalb von 15 Minuten 65,1 ml (0,346 Mol) einer 30proztentigen wässrigen Natriumnitritlösung, rührt anschließend 4 Stunden bei 5 °C, gibt dann bei 10 °C tropfenweise unter Rühren und Kühlung im Verlauf von 2 Stunden eine Lösung aus 180 ml konzentrierter Salzsäure und 205 g (0,35 Mol) Zinndichlorid-Dihydrat zu, rührt eine weitere Stunde bei 10 °C und 15 Stunden bei Raumtemperatur, saugt ab bei 5 °C, nimmt den Rückstand in 300 ml konzentriertem wässrigem Ammoniak auf, rührt 1 Stunde bei Raumtemperatur, saugt wiederum bei 5 °C ab, wäscht den Rückstand dreimal mit jeweils 300 ml Dichlormethan, trocknet die vereinigten organischen Phasen über Magnesiumsulfat und entfernt das Lösungsmittel im Vakuum.

Man erhält 32 g (50 % der Theorie) an 2-Fluor-4,5-Dioxydifluormethylen-phenylhydrazin als Öl.

$^1$H-NMR (Dimethylsulfoxid-$d_6$/Tetramethylsilan):

$\delta$ = 4,15 (s,2H); 6,8 (s, 1H); 7,15 (s,1H); 7,35 (d,1H) ppm.

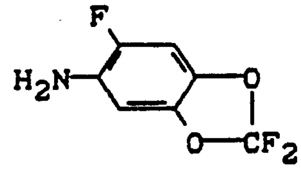

230 g (1,04 Mol) 5-Nitro-2,2,6-trifluor-1,3-benzodioxol und 20 g Raney-Nickel in 800 ml Tetrahydrofuran werden bei 20 °C bis 45 °C und 30 bis 50 bar Wasserstoffdruck bis zum Ende der Wasserstoffaufnahme hydriert. Zur Aufarbeitung wird der Katalysator abfiltiert und das Filtrat destilliert.

Man erhält 179 g (90 % der Theorie) an 5-Amino-2,2,6-trifluor-1,3-benzodioxol vom Siedepunkt 86 °C - 87 °C bei 16 mbar und vom Brechungsindex $n_D^{20}$ 1,4812.

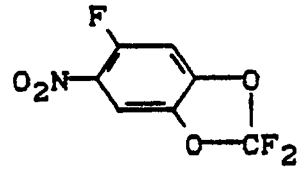

Zu einer Mischung aus 202 ml 67prozentiger Salpetersäure und 242 ml konzentrierter Schwefelsäure tropft man bei 10 °C unter Rühren 202 g (1,15 Mol) 2,2,5-Trifluor-1,3-benzodioxol (vgl. Doklady Akad. Nauk. S.S.S.R. 135, 377-380 [1960] bzw C.A.: 55, 24 238e), rührt nach beendeter Zugabe eine Stunde bei 10 °C und eine weitere Stunde bei 20 °C, gießt die Mischung auf 600 g Eis, trennt die organische Phase ab und destilliert.

Man erhält 232 g (91,3 % der Theorie) an 5-Nitro-2,2,6-trifluor-1,3-benzodioxol vom Siedepunkt 113 °C - 114 °C bei 20 mbar und vom Brechungsindex $n_D^{20}$ 1,4968.

Beispiel VI-1

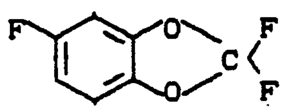

Zu 173 g (1 Mol) 5-Amino-2,2-difluor-1,3-benzodioxol (vgl. z.B. DE 2 823 168; EP 42 533) in 600 ml Wasser und 150 ml konzentrierter Salzsäure gibt man bei 0 °C 70 g (1 Mol) Natriumnitrit in 180 ml Wasser, rührt 1 Stunde bei 0 °C bis 10 °C, filtriert die Reaktionsmischung in 400 ml 30prozentige wässrige Tetrafluorborsäurelösung, saugt den ausgefallenen Niederschlag ab, trocknet bei 50 °C und 20 mbar und erwärmt das so erhältliche Diazoniumtetrafluoroborat portionsweise (in Portionen von ca. 20 bis 30 g) in einer Destillationsapparatur auf 130 °C bis 160 °C. Das Produkt destilliert dabei mit dem Gasstrom ab und wird in einer gut gekühlten Vorlage aufgefangen. Zum Schluß steigert man die Badtemperatur auf 190 °C um die Reaktion und Destillation zu komplettieren, nimmt das überdestillierte Produkt in Dichlormethan auf, wäscht mit Wasser, trocknet über Magnesiumsulfat und destilliert ein zweites Mal über eine Kolonne.

Man erhält 89 g (50 % der Theorie) an 2,2,5-Trifluor-1,3-benzodioxol vom Siedepunkt 128 °C - 130 °C und vom Brechungsindex $n_D^{20}$ 1,4295.

Anwendungsbeispiele

In den folgenden Anwendungsbeispielen wurde die nachstehend aufgeführte Verbindung als Vergleichssubstanz eingesetzt:

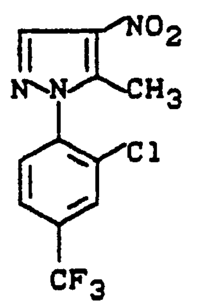

(A)

**1-(2-Chlor-4-trifluormethylphenyl)-5-methyl-4-nitro-pyrazol**

**(bekannt aus EP-A 200 872)**

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzübereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzübereitung begassen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegen mono- und dikotyle Unkräuter ebenso wie in der Nutzpflanzenselektivität, Insbesondere bei Weizen und Soja, gegenüber der Vergleichssubstanz (A) zeigt in diesem Test z.B. die Verbindung gemäß dem Herstellungsbeispiel 1.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzübereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzübereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegen dikotyle Unkräuter ebenso wie in der Nutzpflanzenselektivität, insbesondere bei Weizen, gegenüber der Vergleichssubstanz (A) zeigt in diesem Test z.B. die Verbindung gemäß den Herstellungsbeispiel 1.

## Ansprüche

1. N-Aryl-Stickstoffheterocyclen der allgemeinen Formel (I),

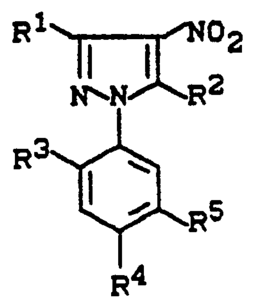

(I)

in welcher
$R^1$ für Wasserstoff, Alkyl oder Halogenalkyl steht,
$R^2$ für Wasserstoff, Alkyl oder Halogenalkyl steht,
$R^3$ für Wasserstoff oder Halogen steht und entweder
$R^4$ für Cyano, Nitro, Alkyl, Alkenyl, Alkinyl, Alkoxy, Halogenalkyl oder Halogenalkoxy steht und
$R^5$ für Halogen, für einen Rest -O-$R^6$ oder für einen Rest -S-$R^7$ steht oder
$R^4$ und $R^5$ gemeinsam für einen gegebenenfalls substituierten Dioxyalkylenrest stehen,
wobei
$R^6$ für Wasserstoff, für jeweils gegebenenfalls substituiertes Alkyl, Alkenyl, Alkinyl oder Cycloalkyl steht oder für einen Rest

$$-\underset{\underset{O}{\|}}{C}-R^8$$

oder einen Rest -$SO_2$-$R^9$ steht,
$R^7$ für Wasserstoff, für jeweils gegebenenfalls substituiertes Alkyl, Alkenyl, Alkinyl oder Cycloalkyl steht oder für einen Rest

$$-\underset{\underset{O}{\|}}{C}-R^8$$

steht,
$R^8$ für Alkyl, Alkoxy oder Halogenalkyl steht und
$R^9$ für Alkyl, Halogenalkyl oder für gegebenenfalls substituiertes Aryl steht,
wobei jedoch die Verbindung 1-(3,4-Dimethoxyphenyl)-4-nitropyrazol ausgenommen ist.

2. N-Aryl-Stickstoffheterocyclen der Formel (I) gemäß Anspruch 1, in welcher
$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^2$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^3$ für Wasserstoff, Fluor, Chlor oder Brom steht und entweder
$R^4$ für Cyano, Nitro, für jeweils geradkettiges oder verzweigtes Alkyl oder Alkoxy mit jeweils 1 bis 4 Kohlenstoffatomen, für jeweils geradkettiges oder verzweigtes Alkenyl oder Alkinyl mit jeweils 2 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen steht und
$R^5$ für Fluor, Chlor, Brom oder für einen Rest -O-$R^6$ oder für einen Rest -S-$R^7$ steht oder
$R^4$ und $R^5$ gemeinsam für einen gegebenenfalls einfach oder mehrfach, gleich oder verschieden durch Fluor, Chlor, Brom oder durch geradkettiges oder verzweigtes Alkyl mit 1 bis 4 Kohlenstoffatomen substituierten Dioxyalkylenrest mit 1 bis 3 Kohlenstoffatomen stehen, wobei
$R^6$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Alkoxyalkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen;

$R^6$ weiterhin für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^6$ ferner für einen Rest $-\underset{\underset{O}{\|}}{C}-R^8$

oder für einen Rest $-SO_2-R^9$ steht,

$R^7$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Alkoxyalkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen;

$R^7$ weiterhin für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^7$ ferner für einen Rest $-\underset{\underset{O}{\|}}{C}-R^8$

steht,

$R^8$ für jeweils geradkettiges oder verzweigtes Alkyl, Alkoxy oder Halogenalkyl mit jeweils 1 bis 6 Kohlenstoffatomen und im Fall des Halogenalkyl mit 1 bis 13 gleichen oder verschiedenen Halogenatomen steht und

$R^9$ für jeweils geradkettiges oder verzweigtes Alkyl oder Halogenalkyl mit jeweils 1 bis 6 Kohlenstoffatomen und im Fall des Halogenalkyl mit 1 bis 13 gleichen oder verschiedenen Halogenatomen steht und außerdem für gegebenenfalls einfach oder mehrfach, gleich oder verschieden substituiertes Phenyl steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy, oder Halogenalkylthio mit jeweils 1 bis 4 Kohlenstoffatomen und 1 bis 9 gleichen oder verschiedenen Halogenatomen

wobei jedoch die Verbindung 1-(3,4-Dimethoxyphenyl)-4-nitropyrazol ausgenommen ist.

3. N-Aryl-Stickstoffheterocyclen der Formel (I) gemäß Anspruch 1, in welcher

$R^1$ für Wasserstoff, Methyl, Ethyl, n- oder i-Propyl, n-, i-, s- oder t-Butyl, Trifluormethyl, Difluormethyl, Fluormethyl, Trichlormethyl, Dichlormethyl oder Chlormethyl steht,

$R^2$ für Wasserstoff, Methyl, Ethyl, n- oder i-Propyl, n-, i-, s- oder t-Butyl, Trifluormethyl, Difluormethyl, Fluormethyl, Trichlormethyl, Dichlormethyl oder Chlormethyl steht,

$R^3$ für Wasserstoff, Fluor oder Chlor steht und entweder

$R^4$ für Cyano, Nitro, Methyl, Methoxy, Ethyl, Ethoxy, Vinyl, Allyl, Ethinyl, Propargyl, Trifluormethyl, Difluormethyl, Trifluormethoxy oder Difluormethoxy steht und

$R^5$ für Fluor oder Chlor, für einen Rest $-O-R^6$ oder für einen Rest $-S-R^7$ steht oder

$R^4$ und $R^5$ gemeinsam für einen gegebenenfalls ein-bis vierfach, gleich oder verschieden durch Fluor, Chlor oder Methyl substituierten Dioxyalkylenrest mit 1 oder 2 Kohlenstoffatomen stehen, wobei

$R^6$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein- bis dreifach, gleich oder verschieden substituiertes Benzyl oder Phenylethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio;

$R^6$ ferner für einen Rest $-\underset{\underset{O}{\|}}{C}-R^8$

oder für einen Rest $-SO_2-R^9$ steht,

$R^7$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein- bis dreifach, gleich oder verschieden substituiertes Benzyl oder Phenylethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio oder

$R^7$ für einen Rest $-\underset{\underset{O}{\|}}{C}-R^8$

steht,

$R^8$ für jeweils geradkettiges oder verzweigtes Alkyl, Alkoxy oder Halogenalkyl mit jeweils 1 bis 3 Kohlenstoffatomen und im Fall des Halogenalkyl mit bis 7 gleichen oder verschiedenen Halogenatomen steht und

$R^9$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach gleich oder verschieden substituiertes Phenyl steht, wobei als Substituenten infrage kommen:

Fluor, Chlor, Brom, Cyano, Nitro, Methyl, Ethyl, n- oder i-Propyl, n-, i, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

wobei jedoch die Verbindung 1-(3,4-Dimethoxyphenyl)-4-nitropyrazol ausgenommen ist.

4. N-Aryl-Stickstoffheterocyclen der Formel (I) gemäß Anspruch 1, in welcher

$R^1$ für Wasserstoff, Methyl, Ethyl oder Trifluormethyl steht,

$R^2$ für Wasserstoff, Methyl, Ethyl oder Trifluormethyl steht,

$R^3$ für Wasserstoff oder Fluor steht und entweder

$R^4$ für Cyano, Nitro, Methyl, Methoxy, Trifluormethyl, Difluormethyl, Trifluormethoxy oder Difluormethoxy steht und

$R^5$ für Fluor oder für einen Rest $-O-R^6$ oder für einen Rest $-S-R^7$ steht oder

$R^4$ und $R^5$ gemeinsam für einen gegebenenfalls einfach oder zweifach durch Fluor, Chlor oder Methyl substituierten Dioxymethylenrest oder für einen gegebenenfalls ein- bis vierfach gleich oder verschieden

durch Fluor, Chlor oder Methyl substituierten Dioxyethylenrest stehen, wobei

$R^6$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein- bis dreifach gleich oder verschieden substituiertes Benzyl oder Phenylethyl steht, wobei als Substituenten jeweils in Frage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio;

$R^6$ ferner für einen Rest $-\underset{\underset{O}{\|}}{C}-R^8$

oder für einen Rest $-SO_2-R^9$ steht,

$R^7$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht oder für jeweils gegebenenfalls ein- bis dreifach gleich oder verschieden substituiertes Benzyl oder Phenylethyl steht, wobei als Substituenten jeweils in Frage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio oder

$R^7$ für einen Rest $-\underset{\underset{O}{\|}}{C}-R^8$

steht,

$R^8$ für jeweils geradkettiges oder verzweigtes Alkyl, Alkoxy oder Halogenalkyl mit jeweils 1 bis 3 Kohlenstoffatomen und im Fall des Halogenalkyl mit 1 bis 7 gleichen oder verschiedenen Halogenatomen steht und

$R^9$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach gleich oder verschieden substituiertes Phenyl steht, wobei als Substituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

wobei jedoch die Verbindung 1-(3,4-Dimethoxyphenyl)-4-nitropyrazol ausgenommen ist.

5. Verfahren zur Herstellung von N-Aryl-Stickstoffheterocyclen der Formel (I)

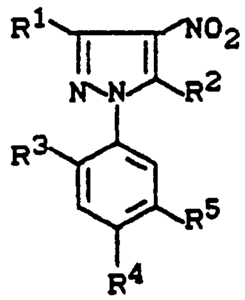

(I)

in welcher
$R^1$ für Wasserstoff, Alkyl oder Halogenalkyl steht,
$R^2$ für Wasserstoff, Alkyl oder Halogenalkyl steht,
$R^3$ für Wasserstoff oder Halogen steht und entweder
$R^4$ für Cyano, Nitro, Alkyl, Alkenyl, Alkinyl, Alkoxy, Halogenalkyl oder Halogenalkoxy steht und
$R^5$ für Halogen, für einen Rest $-O-R^6$ oder für einen Rest $-S-R^7$ steht oder
$R^4$ und $R^5$ gemeinsam für einen gegebenenfalls substituierten Dioxyalkylenrest stehen,
wobei
$R^6$ für Wasserstoff, für jeweils gegebenenfalls substituiertes Alkyl, Alkenyl, Alkinyl oder Cycloalkyl steht oder für einen Rest
$-\underset{\underset{O}{\|}}{C}-R^8$
oder einen Rest $-SO_2-R^9$ steht,
$R^7$ für Wasserstoff, für jeweils gegebenenfalls substituiertes Alkyl, Alkenyl, Alkinyl oder Cycloalkyl steht oder für einen Rest $-\underset{\underset{O}{\|}}{C}-R^8$
steht,
$R^8$ für Alkyl, Alkoxy oder Halogenalkyl steht und
$R^9$ für Alkyl, Halogenalkyl oder für gegebenenfalls substituiertes Aryl steht,
wobei jedoch die Verbindung 1-(3,4-Dimethoxyphenyl)-4-nitropyrazol ausgenommen ist,
dadurch gekennzeichnet, daß man

(a) N-Aryl-Stickstoffheterocyclen der Formel (I),

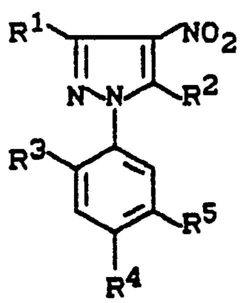

(I)

in welcher
$R^1$, $R^2$, $R^3$, $R^4$ und $R^5$ die oben angegebene Bedeutung haben,
erhält, wenn man 4-unsubstituierte 1-Phenylpyrazole der Formel (II),

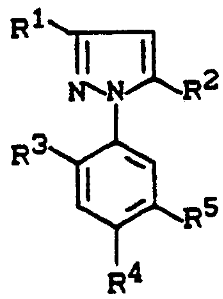

(II)

in welcher
$R^1$, $R^2$, $R^3$, $R^4$ und $R^5$ die oben angegebene Bedeutung haben,
mit einem Nitrierungsmittel gegebenenfalls in Gegenwart eines Verdünnungsmittels und gegebenenfalls in Gegenwart eines Reaktionshilfsmittels umsetzt; oder daß man

(b) N-Aryl-Stickstoffheterocyclen der Formel (Ia),

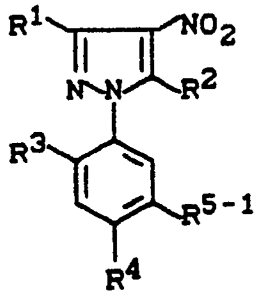

(Ia)

in welcher
$R^{5-1}$ für einen Rest $-O-R^{6-1}$ oder für einen Rest $-S-R^{7-1}$ steht, wobei
$R^{6-1}$ für jeweils gegebenenfalls substituiertes Alkyl, Alkenyl, Alkinyl oder Cycloalkyl steht, für einen Rest $-\underset{\underset{O}{\|}}{C}-R^8$ oder für einen Rest $-SO_2-R^9$ steht und
$R^{7-1}$ für jeweils gegebenenfalls substituiertes Alkyl, Alkenyl, Alkinyl oder Cycloalkyl steht oder für einen Rest $-\underset{\underset{O}{\|}}{C}-R^8$ steht
und wobei
$R^1$, $R^2$, $R^3$, $R^4$, $R^8$ und $R^9$ die oben angegebene Bedeutung haben,
erhält, wenn man N-Aryl-Stickstoffheterocyclen der Formel (Ib),

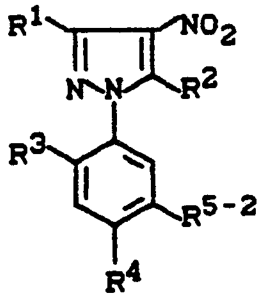

(Ib)

in welcher
$R^{5-2}$ für Hydroxy oder Mercapto steht und
$R^1$, $R^2$, $R^3$ und $R^4$ die oben angegebene Bedeutung haben,

(α) mit Alkylierungsmitteln der Formel (IIIa),

$R^{10}-E^1$ (IIIa)

in welcher

$R^{10}$ für jeweils gegebenenfalls substituiertes Alkyl, Alkenyl, Akinyl oder Cycloalkyl steht und

$E^1$ für eine elektronenanziehende Abgangsgruppe steht oder

(β) mit Acylierungsmitteln der Formel (IIIb), $R^8-\underset{\underset{O}{\|}}{C}-E^2$ (IIIb)

in welcher

$E^2$ für eine elektronenanziehende Abgangsgruppe steht und

$R^8$ die oben angegebene Bedeutung hat und

(γ) mit Sulfonylierungsmitteln der Formel (IIIc),

$R^9-SO_2-E^3$ (IIIc)

in welcher

$E^3$ für eine elektronenanziehende Abgangsgruppe steht und

$R^9$ die oben angegebene Bedeutung hat,

jeweils gegebenenfalls in Gegenwart eines Verdünnungsmittels und gegebenenfalls in Gegenwart eines Reaktionshilfsmittels umsetzt; oder daß man

(c) N-Aryl-Stickstoffheterocyclen der Formel (Ic),

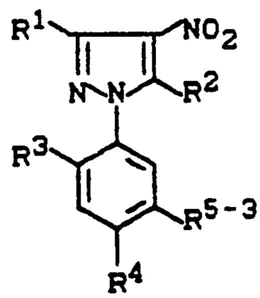

(Ic)

in welcher

$R^{5-3}$ für einen Rest -O-$R^{10}$ oder für einen Rest -S-$R^{10}$ steht wobei

$R^{10}$ für jeweils gegebenenfalls substituiertes Alkyl, Alkenyl, Alkinyl oder Cycloalkyl steht und wobei

$R^1$, $R^2$, $R^3$ und $R^4$ die oben angegebene Bedeutung haben,

erhält, wenn man N-Aryl-Stickstoffheterooyolen der Formel (Id),

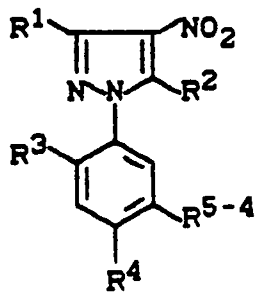

(Id)

in welcher

$R^{5-4}$ für Halogen steht und

$R^1$, $R^2$, $R^3$ und $R^4$ die oben angegebene Bedeutung haben,

mit Alkoholen oder Thiolen der Formel (IV),

$R^{5-3}-H$ (IV)

in welcher

$R^{5-3}$ die oben angegebene Bedeutung hat,

gegebenenfalls in Gegenwart eines Verdünnungsmittels und gegebenenfalls in Gegenwart eines Reaktionshilfsmittels umsetzt; oder daß man

N-Aryl-Stickstoffheterocyclen der Formel (Ie),

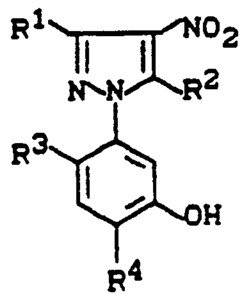

(Ie)

in welcher
$R^1$, $R^2$, $R^3$ und $R^4$ die oben angegebene Bedeutung haben,
erhält, wenn man

(d) N-Aryl-Stickstoffheterocyclen der Formel (If) ,

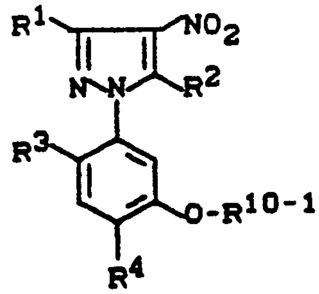

(If)

in welcher
$R^{10-1}$ für Allyl oder für Benzyl steht,
entweder für den Fall, daß $R^{10-1}$ für Benzyl steht mit Wasserstoff in Gegenwart eines geeigneten Hydrierkatalysators oder für den Fall, daß $R^{10-1}$ für Allyl steht mit verdünnten Säuren in Gegenwart eines geeigneten Isomerisierungskatalysators sowie jeweils gegebenenfalls in Gegenwart eines Verdünnungsmittels und gegebenenfalls in Gegenwart eines Reaktionshilfsmittels umsetzt; oder daß man

(e) N-Aryl-Stickstoffheterocyclen der Formel (Ig),

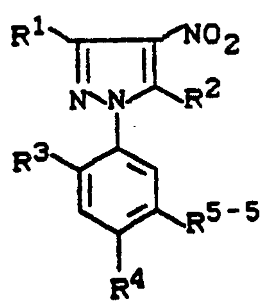

(Ig)

in welcher
$R^{5-5}$ für einen Rest $-O-\underset{\underset{O}{\|}}{C}-R^8$
oder für einen Rest $-O-SO_2-R^9$ steht und
$R^1$, $R^2$, $R^3$ und $R^4$ die oben angegebene Bedeutung haben,
mit verdünnten Säuren oder Basen gegebenenfalls in Gegenwart eines Verdünnungsmittels umsetzt; oder daß man

(f) N-Aryl-Stickstoffheterocyclen der Formel (Ih),

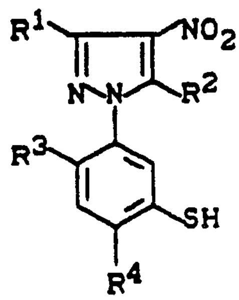

(Ih)

in welcher
$R^1$, $R^2$, $R^3$ und $R^4$ die oben angegebene Bedeutung haben,
erhält, wenn man N-Arylstickstoffheterocyclen der Formel (Id),

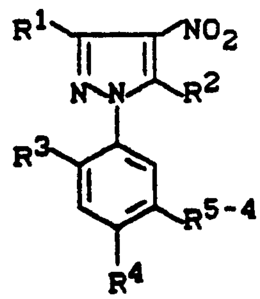

(Id)

in welcher
$R^{5-4}$ für Halogen steht und
$R^1$, $R^2$, $R^3$ und $R^4$ die oben angegebene Bedeutung haben,
mit Natriumhydrogensulfid gegebenenfalls in Gegenwart eines Verdünnungsmittels und gegebenenfalls in Gegenwart eines Reaktionshilfsmittels umsetzt.

6. Herbizide Mittel, gekennzeichnet durch einen Gehalt an mindestens einem N-Aryl-Stickstoffheterocyclus der Formel (I) gemäß den Ansprüchen 1 bis 5.

7. Verfahren zur Bekämpfung von Unkräutern, dadurch gekennzeichnet, daß man N-Aryl-Stickstoffheterocyclen der Formel (I) gemäß den Ansprüchen 1 bis 5 auf die Unkräuter und/oder ihren Lebensraum einwirken läßt.

8. Verwendung von N-Aryl-Stickstoffheterocyclen der Formel (I) gemäß den Ansprüchen 1 bis 5 zur Bekämpfung von Unkräutern.

9. Verfahren zur Herstellung von herbiziden Mitteln dadurch gekennzeichnet, daß man N-Aryl-Stickstoffheterocyclen der Formel (I) gemäß den Ansprüchen 1 bis 5 mit Streckmitteln und/oder oberflächenaktiven Substanzen vermischt.

Europäisches Patentamt

# EUROPÄISCHER RECHERCHENBERICHT

Nummer der Anmeldung

EP 90 10 1681

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 200 872 (BAYER AG)<br>* Das ganze Dokument *<br>--- | 1-9 | C 07 D 231/16<br>A 01 N 43/56<br>C 07 D 405/04 |
| A | EP-A-0 202 169 (HOKKO CHEMICAL IND. CO., LTD)<br>----- | | |

| RECHERCHIERTE SACHGEBIETE (Int. Cl.5) |
|---|
| C 07 D 231/00<br>A 01 N 43/00<br>C 07 D 405/00 |

Der vorliegende Recherchenbericht wurde für alle Patentansprüche erstellt

| Recherchenort | Abschlußdatum der Recherche | Prüfer |
|---|---|---|
| DEN HAAG | 11-05-1990 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument